# EUROPEAN PATENT SPECIFICATION

(11) **EP 1 838 345 B1**
(45) Date of publication and mention of the grant of the patent: **22.12.2010**
(21) Application number: 05850768.2
(22) Date of filing: 23.12.2005
(51) Int. Cl.: A61K 39/385, A61K 39/116, A61K 39/095, A61P 37/04

(54) **SACCHARIDE CONJUGATE VACCINES**
SACCHARID-KONJUGATVAKZINE
VACCINS A BASE DE CONJUGUES DE SACCHARIDE

(30) Priority: 24.12.2004 GB 0428394
(43) Date of publication of application: 03.10.2007
(73) Proprietor: Novartis Vaccines and Diagnostics S.r.l., 53100 Siena (SI) (IT)
(72) Inventor: DEL GIUDICE, Giuseppe, Chiron Srl, 53100 Siena (IT); BARALDO, Karin, 39100 Bolzano (IT)
(74) Representative: Marshall, Cameron John
(86) International application number: PCT/IB2005/004050
(87) International publication number: WO 2006/067632

(56) References cited:
- WO-A-98/43677
- WO-A-99/55730
- BARALDO KARIN ET AL: "N19 polyepitope as a carrier for enhanced immunogenicity and protective efficacy of meningococcal conjugate vaccines." INFECTION AND IMMUNITY. AUG 2004, vol. 72, no. 8, August 2004 (2004-08), pages 4884-4887, XP002401048 ISSN: 0019-9567
- FALUGI F ET AL: "Rationally designed strings of promiscuous CD4(+) T cell epitopes provide help to Haemophilus influenzae type b oligosaccharide: a model for new conjugate vaccines." EUROPEAN JOURNAL OF IMMUNOLOGY. DEC 2001, vol. 31, no. 12, December 2001 (2001-12), pages 3816-3824, XP002401049 ISSN: 0014-2980
- BALMER P ET AL: "Impact of meningococcal C conjugate vaccine in the UK." JOURNAL OF MEDICAL MICROBIOLOGY. SEP 2002, vol. 51, no. 9, September 2002 (2002-09), pages 717-722, XP002401050 ISSN: 0022-2615
- BARALDO KARIN ET AL: "Combined conjugate vaccines: enhanced immunogenicity with the N19 polyepitope as a carrier protein." INFECTION AND IMMUNITY. SEP 2005, vol. 73, no. 9, September 2005 (2005-09), pages 5835-5841, XP002401051 ISSN: 0019-9567

## Description

### TECHNICAL FIELD

This invention is in the field of vaccines and relates to new compositions comprising two or more saccharide antigens conjugated to a polyepitope carrier protein comprising T cell epitopes from multiple pathogenic proteins. The invention also relates to methods for making said compositions and to uses for said compositions.

### BACKGROUND ART

Polyvalent vaccines are known in the art. One such example is a tetravalent vaccine of capsular polysaccharides from serogroups A, C, Y and W135 of *N. meningitidis* which has been known for many years [1, 2] and has been licensed for human use. However, although effective in adolescents and adults, it induces a poor immune response and short duration of protection and cannot be used in infants [*e.g*. 3]. This is because polysaccharides are T cell-independent antigens that generally induce a weak immune response that cannot be boosted. Concerns have often arisen regarding the widespread use of polyvalent vaccines because they are subject to a significant decrease in immune function known as immunosuppression. Immunosuppression may result when the amount of antigen introduced into the subject exceeds the ability of the immune system to respond. Such a condition is termed antigen-overload. Immunosuppression may also occur as a result of one antigen component preventing the immune system from responding to another antigen component of a polyvalent vaccine. This latter form of immunosuppression is termed vaccine interference.

In the last 20 years, conjugate vaccines, comprising bacterial capsular polysaccharides conjugated to protein carriers have developed. Examples include the *Haemophilus influenzae* type b (Hib) conjugate vaccine [4] as well as conjugate vaccines against *Streptococcus pneumoniae* [5] and serogroup C *Neisseria meningitidis* (MenC) [6].

The carrier proteins used in licensed vaccines include tetanus toxoid (TT), diphtheria toxoid (DT), the nontoxic CRM197 mutant of diptheria toxin, and the outer membrane protein complex from group B *N.meningitidis.* Since more conjugated vaccines are being introduced into the medical practice, infants could receive multiple injections of the carrier protein, either as a vaccine itself (*e.g*. TT or DT) or as a carrier protein present in a conjugate vaccine. As these proteins are highly immunogenic at both the B- and T-cell level, carrier overload may induce immune suppression in primed individuals [7]. This phenomenon, termed carrier-induced epitopic suppression, is thought to be due to carrier specific antibodies and intramolecular antigenic competition [8]. Ideally, a carrier protein should induce strong helper effect to a conjugated B-cell epitope (*e.g*. polysaccharide) without inducing an antibody response against itself. The use of universal epitopes, which are immunogenic in the context of most major histocompatability complex class II molecules, is one approach towards this goal [9]. Such epitopes have been identified within TT and other proteins. However, there remains the need for further improvements.

It is therefore the object of the invention to provide improved saccharide conjugates.

### DISCLOSURE OF THE INVENTION

It has been discovered that polyepitope carrier proteins are particularly useful as carriers for combinations of saccharides. Furthermore, it has been discovered that only a low immunogenic response is seen against these carrier proteins even though they comprise a number of known pathogenic epitopes, whereas it would have been expected that the immunogenic response would increase proportionally to the number of pathogenic epitopes.

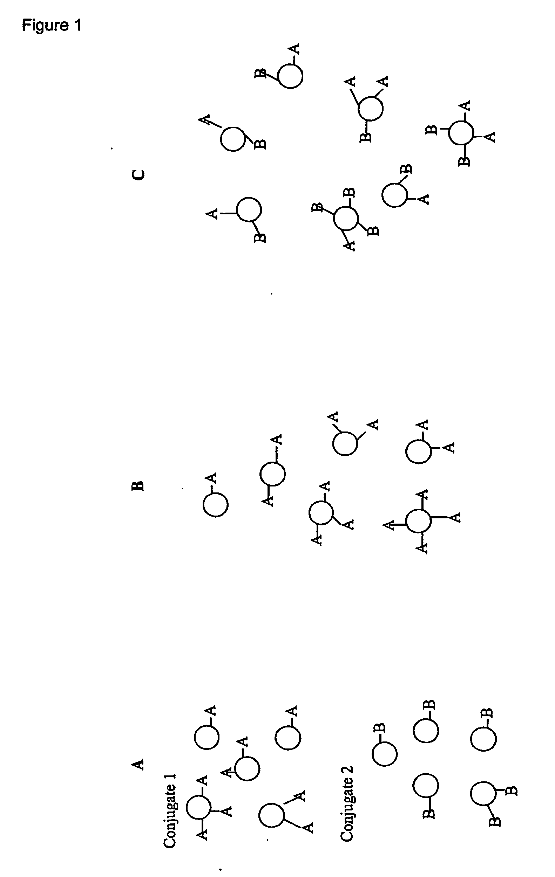

In some embodiments, the invention therefore provides a composition comprising a combination of two or more monovalent conjugates (*e.g*. 2, 3, 4, 5, 6 or more. See Figure 1A). Each monovalent conjugate comprises (i) N19 carrier protein conjugated to (ii) a saccharide antigen from *N.meningitidis* serogroup A, C, W, or Y. Preferably the carrier protein used in each conjugate is the same.

Although each carrier protein molecule in each monovalent conjugate may be conjugated to more than one saccharide antigen molecule (*e.g*. 1, 5, 10, 20 or more) due to the multiple attachment sites on each carrier protein molecule (Figure 1B), each saccharide antigen conjugated to any given carrier protein is preferably from the same antigenically distinct pathogen. For example, saccharide antigens from MenA are different from those from each of MenC, MenW and MenY and are therefore said to be from antigenically distinct pathogens, whereas saccharide antigens from Hib are all from the same antigenically distinct pathogen. In a single conjugate, individual saccharides, although from the same antigenically distinct pathogen, may be of different chain lengths.

As an alternative, in some embodiments, the invention provides a multivalent conjugate comprising two or more (*e.g*. 2, 3, 4, 5, 6 or more) antigenically distinct saccharide antigens from N.meningitidis serogroups A, C, W, or Y conjugated to N19 carrier protein (Figure 1C). In this case, the saccharide antigens are from different antigenically distinct pathogens. Therefore, for example, in such a conjugate composition, each N19 carrier protein molecule may have saccharide antigens from two or more of MenA, MenC, MenW, MenY conjugated to it. The invention also provides a composition comprising two or more (*e.g*. 2, 3, 4, 5, 6 or more) of these multivalent conjugates.

As a further alternative, the invention provides a composition comprising one or more (*e.g*. 1, 2, 3, 4, 5, 6 or more) monovalent conjugate(s) and one or more (*e.g*. 1, 2, 3, 4, 5, 6 or more) multivalent conjugate(s) as described above.

### Carrier protein

The carrier protein is N19 (SEQ ID NO: 11). It has been shown that a genetically engineered protein, termed N19 [10], expressed in *Escherichia coli* and having several human CD4⁺ T-cell universal epitopes, behaves as a strong carrier when conjugated to Hib polysaccharide [11] WO 99/55730A and Falusi, F. et al (2001) Eur J. Immunol, 31(12) 3816-3824, and menc (Baraldo, K.et al (2004) infect immun, 72(8) 4884-4887) . The N-tenminal region of the N19 consists of (i) a six His tail that may be exploited during purification, (ii) a flag peptide Met-Asp-Tyr-Lys-Asp-Asp-Asp-Asp sequence (SEQ ID NO: 12) recognized by a rabbit polyclonal antibody that can be used for the screening of positive colonies during the cloning procedure, (iii) the Ile-Glu-Gly-Arg (SEQ ID NO: 13) Factor Xa recognition site for ready elimination of the tag. N19 is a duplication of the first nine epitopes listed in Table 1 plus the influenza matrix CD4⁺ epitope MT. The epitopes are separated by a Lys-Gly spacer to provide flexibility to the molecule and to allow the subsequent conjugation of the polysaccharide to the primary ε-amino groups of Lys residues.

In addition to CD4⁺ epitopes, carrier proteins may comprises other peptides or protein fragments, such as epitopes from immunomodulating cytokines such as interleukin-2 (IL-2) or granulocyte-macrophage colony stimulating factor (GM-CSF).

**Table 1**

| **T-cell epitope** | **Origin** | **aa position** | **Amino acid sequence (SEQ ID NO)** | **References** |
|---|---|---|---|---|
| **P23TT** | Tetanus toxin | 1084-1099 | VSIDKFRIFCKANPK (1) | 12 |
| **P32TT** | Tetanus toxin | 1174-1189 | LKFIIKRYTPNNEIDS (2) | 12 |
| **P21TT** | Tetanus toxin | 1064-1079 | IREDNNITLKLDRCNN (3) | 13 |
| **PfCs** | *P.falciparum* Circumsporozoite protein | 380-398 | EKKIAKMEKASSVFNVVN (4) | 14 |
| **P30TT** | Tetanus toxin | 947-967 | FNNFTVSFWLRVPKVSASHLE | 15 |
| **P2TT** | Tetanus toxin | 830-843 | QYIKANSKFIGITE (6) | 15,16. |
| **HBVnc** | Hepatitis B virus Nucleocapsid | 50-69 | PHHTALRQAlLCWOELMTLA (7) | 17 |
| **HA** | Influenza virus Hemagglutinin | 307-319 | PKYVKQNTLKLAT(8) | 16 |
| **HBsAg** | Hepatitis B virus Surface protein | 19-33 | FFLLTRILTIPQSLD (9) | 18 |
| **MT** | Influenza virus Matrix protein | 17-31 | YSGPLKAEIAQRLEDV (10) | 19 |

### Saccharide antigens

The saccharide antigen conjugated to the carrier protein in a composition of the invention is a bacterial capsular saccharide from *Neisseria meningitidis* (serogroups A, C, W135 and/or Y),

The *N.meningitidis* serogroup A (MenA) capsule is a homopolymer of (α1→6)-linked *N*-acetyl-D-mannosamine-1-phosphate, with partial O-acetylation in the C3 and C4 positions. The *N.meningitidis* serogroup C (MenC) capsular saccharide is a homopolymer of (α 2→9) linked sialic acid, with variable O-acetylation at positions 7 and/or 8. The *N.meningitidis* serogroup W135 saccharide is a polymer having sialic acid-galactose disaccharide units [→4)-D-Neu*p*5Ac(7/90Ac)-α-(2-6)-D-Gal-α-(1→], It has variable O-acetylation at the 7 and 9 positions of the sialic acid [24]. The *N.meningitidis* serogroup Y saccharide is similar to the serogroup W135 saccharide, except that the disaccharide repeating unit includes glucose instead of galactose [→4)-D-Neu*p*5Ac(7/9OAc)-α-(2→6)-D-Glc-α-(1→]. It also has variable O-acetylation at positions 7 and 9 of the sialic acid.

The compositions of the invention comprise mixtures of saccharide antigens. Preferably the compositions comprise 2, 3, 4 or more different saccharide antigens. The antigens may be from the same or from antigenically distinct pathogens. Compositions of the invention comprise saccharide antigens from more than one serogroup of *N. meningitidis. e.g.* compositions may comprise saccharides conjugates from serogroups A+C, A+W135, A+Y, C±W135, C+Y, W135+Y, A+C+W135, A+C+Y, C+W135+Y, A+C+W135+Y, *etc.* Preferred compositions comprise saccharides from serogroups C and Y. Other preferred compositions comprise saccharides from serogroups C, W135 and Y. Particularly preferred compositions comprise saccharides from serogroups A, C, W135 and Y.

Where a mixture comprises meningococcal saccharides from serogroup A and at least one other serogroup saccharide, the ratio (w/w) of MenA saccharide to any other serogroup saccharide may be greater than 1 (*e.g.* 2:1, 3:1, 4:1, 5:1, 10:1 or higher). Ratios between 1:2 and 5:1 are preferred, as are ratios between 1:1.25 and 1:2.5. Preferred ratios (w/w) for saccharides from serogroups A:C:W135:Y are: 1:1:1:1; 1:1:1:2; 2:1:1:1; 4:2:1:1; 8:4:2:1; 4:2:1:2; 8:4:1:2; 4:2:2:1; 2:2: 1: 1; 4:4:2: 1; 2:2:1:2; 4:4:1:2; and 2:2:2: 1.

The invention also provides, in some embodiments, combinations of conjugates where the carrier protein is not the same for each conjugate.

Further preferred compositions of the invention comprise a first conjugate and a second conjugate. The first conjugate comprises a saccharide antigen from N.meningitidis serogroup A, C, W, or Y, conjugated to N19 carrier protein; and the second conjugate comprises a saccharide antigen from N.meningitidis serogroup A, C, W, or Y, conjugated to a carrier protein different from N19. For example the second conjugate may be a N.meningitidis saccharide antigen conjugated to the carrier CRM197. The N.meningitidis saccharide antigen(s) in the second conjugate may be the same as or different from the saccharide antigen(s) in the first conjugate.

### Preparation of capsular saccharide antigens

Methods for the preparation of capsular saccharide antigens are well known. For example, ref. 25 describes the preparation of saccharide antigens from *N. meningitidis* Capsular saccharides can be purified by known techniques, as described in several references herein.

The saccharide antigens may be chemically modified. For instance, they may be modified to replace one or more hydroxyl groups with blocking groups. This is particularly useful for meningococcal serogroup A where the acetyl groups may be replaced with blocking groups to prevent hydrolysis [29]. Such modified saccharides are still serogroup A saccharides within the meaning of the present invention.

The saccharide may be chemically modified relative to the capsular saccharide as found in nature. For example, the saccharide may be de-O-acetylated (partially or fully), de-N-acetylated (partially or fully), N-propionated (partially or fully), *etc.* De-acetylation may occur before, during or after conjugation, but preferably occurs before conjugation. Depending on the particular saccharide, de-acetylation may or may not affect immunogenicity *e.g*. the NeisVac-C™ vaccine uses a de-O-acetylated saccharide, whereas Menjugate™ is acetylated, but both vaccines are effective. The effect of de-acetylation *etc.* can be assessed by routine assays.

Capsular saccharides may be used in the form of oligosaccharides. These are conveniently formed by fragmentation of purified capsular polysaccharide (*e.g*. by hydrolysis), which will usually be followed by purification of the fragments of the desired size. Fragmentation of polysaccharides is preferably performed to give a final average degree of polymerisation (DP) in the oligosaccharide of less than 30. DP can conveniently be measured by ion exchange chromatography or by colorimetric assays [30].

If hydrolysis is performed, the hydrolysate will generally be sized in order to remove short-length oligosaccharides [31]. This can be achieved in various ways, such as ultrafiltration followed by ion-exchange chromatography. Oligosaccharides with a degree of polymerisation of less than or equal to about 6 are preferably removed for serogroup A *meningococcus,* and those less than around 4 are preferably removed for serogroups W 13 5 and Y.

### Carrier-Saccharide Conjugates

Conjugates of the invention may include small amounts of free (*i.e*. unconjugated) carrier. When a given carrier protein is present in both free and conjugated form in a composition of the invention, the unconjugated form is preferably no more than 5% of the total amount of the carrier protein in the composition as a whole, and more preferably present at less than 2% (by weight).

After conjugation, free and conjugated saccharides can be separated. There are many suitable methods, including hydrophobic chromatography, tangential ultrafiltration, diafiltration *etc*. [see also refs. 32 & 33, *etc.*]*.*

Any suitable conjugation reaction can be used, with any suitable linker where necessary. Attachment of the saccharide antigen to the carrier is preferably via a -NH₂ group *e.g*. in the side chain of a lysine residue in a carrier protein, or of an arginine residue. Where a saccharide has a free aldehyde group then this can react with an amine in the carrier to form a conjugate by reductive amination. Attachment may also be via a -SH group *e.g*. in the side chain of a cysteine residue. Alternatively the saccharide antigen may be attached to the carrier via a linker molecule.

The saccharide will typically be activated or functionalised prior to conjugation. Activation may involve, for example, cyanylating reagents such as CDAP (*e.g*. 1-cyano-4-dimethylamino pyridinium tetrafluoroborate [34, 35, *etc.*]*).* Other suitable techniques use carbodiimides, hydrazides, active esters, norborane, p-nitrobenzoic acid, N-hydroxysuccinimide, S-NHS, EDC, TSTU (see also the introduction to reference 36).

### Linkers

Linkages via a linker group may be made using any known procedure, for example, the procedures described in references 37 and 38. One type of linkage involves reductive amination of the saccharide, coupling the resulting amino group with one end of an adipic acid linker group, and then coupling the carrier protein to the other end of the adipic acid linker group [39, 40]. Other linkers include B-propionamido [41], nitrophenyl-ethylamine [42], haloacyl halides [43], glycosidic linkages [44], 6-aminocaproic acid [45], ADH [46], C4 to C12 moieties [47] *etc.* As an alternative to using a linker, direct linkage can be used. Direct linkages to the protein may comprise oxidation of the polysaccharide followed by reductive amination with the protein, as described in, for example, references 48 and 49.

A process involving the introduction of amino groups into the saccharide (*e.g*. by replacing terminal =O groups with -NH₂) followed by derivatisation with an adipic diester (*e.g*. adipic acid N-hydroxysuccinimido diester) and reaction with carrier protein is preferred.

A bifunctional linker may be used to provide a first group for coupling to an amine group in the saccharide and a second group for coupling to the carrier (typically for coupling to an amine in the carrier).

The first group in the bifunctional linker is thus able to react with an amine group (-NH₂) on the saccharide. This reaction will typically involve an electrophilic substitution of the amine's hydrogen. The second group in the bifunctional linker is able to react with an amine group on the carrier. This reaction will again typically involve an electrophilic substitution of the amine.

Where the reactions with both the saccharide and the carrier involve amines then it is preferred to use a bifunctional linker of the formula X-L-X, where: the two X groups are the same as each other and can react with the amines; and where L is a linking moiety in the linker. A preferred X group is N-oxysuccinimide. L preferably has formula L'-L²-L', where L' is carbonyl. Preferred L² groups are straight chain alkyls with 1 to 10 carbon atoms (*e.g*. C₁, C₂, C₃, C₄, C₅, C₆, C₇, C₈, C₉, C₁₀) *e.g* -(CH₂)₄-.

Other X groups are those which form esters when combined with HO-L-OH, such as norborane, p-nitrobenzoic acid, and sulfo-N-hydroxysuccinimide.

Further bifunctional linkers for use with the invention include acryloyl halides (*e.g*. chloride) and haloacylhalides.

The linker will generally be added in molar excess to modified saccharide.

After conjugation, free and conjugated saccharides can be separated. There are many suitable methods, including hydrophobic chromatography, tangential ultrafiltration, diafiltration *etc.* [see also refs. 50 & 51, *etc.*]*.*

Where the composition of the invention includes a depolymerised saccharide, it is preferred that depolymerisation precedes conjugation.

### Further antigens

Compositions of the invention may comprise one or more (*e.g*. 2, 3, 4, 5, 6, 7, 8, 9, 10 or more) further antigens, such as:

### A. Bacterial Antigens

Bacterial antigens suitable for use in the invention include proteins, polysaccharides, lipopolysaccharides, and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigens examples identified below.

*Neisseria meningitidis:* meningococcal antigens may include proteins (such as those identified in references 52-58), saccharides (including a polysaccharide, oligosaccharide or lipopolysaccharide), or outer-membrane vesicles [59-62] purified or derived from a *N.meningitidis* serogroup such as A, C, W135, Y, and/or B. Meningococcal protein antigens may be selected from adhesins, autotransporters, toxins, iron acquisition proteins, and membrane associated proteins (preferably integral outer membrane proteins). See also refs. 63-71.

*Streptococcus pneumoniae: S.pneumoniae* antigens may include a saccharide (including a polysaccharide or an oligosaccharide) and/or protein from *S.pneumoniae.* Protein antigens may be selected, for example, from a protein identified in any of refs. 72-77. *S.pneumoniae* proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp101, Sp130, Sp125 or Sp133. See also refs. 78-84.

*Streptococcus pyogenes* (Group A Streptococcus): Group A Streptococcus antigens may include a protein identified in reference 85 or 86 (including GAS40), fusions of fragments of GAS M proteins (including those described in refs. 87-89), fibronectin binding protein (Sfb1), Streptococcal heme-associated protein (Shp), and Streptolysin S (SagA). See also refs. 85, 90 and 91.

*Moraxella catarrhalis:* Moraxella antigens include antigens identified in refs. 92 & 93, outer membrane protein antigens (HMW-OMP), C-antigen, and/or LPS. See also ref. 94.

*Bordetella pertussis:* Pertussis antigens include petussis holotoxin (PT) and filamentous haemagglutinin (FHA) from *B.pertussis,* optionally also in combination with pertactin and/or agglutinogens 2 and 3 antigen. See also refs. 95 & 96.

*Staphylococcus aureus: S.aureus* antigens include *S. aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin). See also ref. 97.

*Staphylococcus epidermis: S.epidermidis* antigens include slime-associated antigen (SAA).

*Clostridium tetani* (Tetanus): Tetanus antigens include tetanus toxoid (TT), preferably used as a carrier protein in conjunction/conjugated with the compositions of the present invention.

*Corynebacterium diphtheriae* (Diphtheria): Diphtheria antigens include diphtheria toxin or detoxified mutants thereof, such as CRM197. Additionally antigens capable of modulating, inhibiting or associated with ADP ribosylation are contemplated for combination/coadministration/conjugation with the compositions of the present invention. These diphtheria antigens may be used as carrier proteins.

*Haemophilus influenzae: H.influenzae* antigens include a saccharide antigen from type B, or protein D [98].

*Pseudomonas aeruginosa:* Pseudomonas antigens include endotoxin A, Wzz protein and/or Outer Membrane Proteins, including Outer Membrane Proteins F (OprF) [99].

*Legionella pneumophila.* Bacterial antigens may be derived from *Legionella pneumophila.*

*Streptococcus agalactiae* (Group B Streptococcus): Group B Streptococcus antigens include protein antigens identified in refs. 85 and 100-103. For example, the antigens include proteins GBS80, GBS104, GBS276 and GBS322.

*Neisseria gonorrhoeae:* Gonococcal antigens include Por (or porin) protein, such as PorB [104], a transferring binding protein, such as TbpA and TbpB [105], an opacity protein (such as Opa), a reduction-modifiable protein (Rmp), and outer membrane vesicle (OMV) preparations [106]. See also refs. 52-54 & 107.

*Chlamydia trachomatis: C.trachomatis* antigens include antigens derived from serotypes A, B, Ba and C (agents of trachoma, a cause of blindness), serotypes L₁, L₂ & L₃ (associated with Lymphogranuloma venereum), and serotypes, D-K. *C.trachomatis* antigens may also include an antigen identified in refs. 103 & 108-110, including PepA (CT045), LcrE (CT089), ArtJ (CT381), DnaK (CT396), CT398, OmpH-like (CT242), L7/L12 (CT316), OmcA (CT444), AtosS (CT467), CT547, Eno (CT587), HrtA (CT823), and MurG (CT761). See also ref. 111.

*Treponema pallidum* (Syphilis): Syphilis antigens include TmpA antigen.

*Haemophilus ducreyi* (causing chancroid): Ducreyi antigens include outer membrane protein (DsrA).

*Enterococcus faecalis or Enterococcus faecium:* Antigens include a trisaccharide repeat or other *Enterococcus* derived antigens provided in ref. 112.

*Helicobacter pylori: H.pylori* antigens include Cag, Vac, Nap, HopX, HopY and/or urease antigen. [113-123].

*Staphylococcus saprophyticus:* Antigens include the 160 kDa hemagglutinin of *S.saprophyticus* antigen.

*Yersinia enterocolitica* Antigens include LPS [124].

*Escherichia coli: E.coli* antigens may be derived from enterotoxigenic *E.coli* (ETEC), enteroaggregative *E.coli* (EAggEC), diffusely adhering *E.coli* (DAEC), enteropathogenic *E.coli* (EPEC), and/or enterohemorrhagic *E. coli* (EHEC) strains.

*Bacillus anthracis* (anthrax): *B.anthracis* antigens are optionally detoxified and may be selected: from A-components (lethal factor (LF) and edema factor (EF)), both of which can share a common B-component known as protective antigen (PA). See refs. 125-127.

*Yersinia pestis* (plague): Plague antigens include F1 capsular antigen [128], LPS [129],V antigen [130].

*Mycobacterium tuberculosis:* Tuberculosis antigens include lipoproteins, LPS, BCG antigens, a fusion protein of antigen 85B (Ag85B) and/or ESAT-6 optionally formulated in cationic lipid vesicles [131], *Mycobacterium tuberculosis* (Mtb) isocitrate dehydrogenase associated antigens [132], and/or MPT51 antigens [133].

*Rickettsia:* Antigens include outer membrane proteins, including the outer membrane protein A and/or B (OmpB) [134], LPS, and surface protein antigen (SPA) [135].

*Listeria monocytogenes:* Bacterial antigens may be derived from *Listeria monocytogenes.*

*Chlamydia pneumoniae:* Antigens include those identified in refs. 108 & 136 to 141.

*Vibrio cholerae:* Antigens include proteinase antigens, particularly lipopolysaccharides of *Vibrio cholerae* II, O1 Inaba O-specific polysaccharides, *V. cholera* O139, antigens of IEM108 vaccine [142], and/or *Zonula occludens* toxin (Zot).

*Salmonella typhi* (typhoid fever): Antigens include capsular polysaccharides preferably conjugates (Vi, *e.g*. vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins, such as antigens associated with P39 and P13 (an integral membrane protein, [143]) and VIsE Antigenic Variation Protein [144].

*Porphyromonas gingivalis:* Antigens include the outer membrane protein (OMP). See also ref. 145.

*Klebsiella:* Antigens include an OMP, including OMP A, or a polysaccharide optionally conjugated to tetanus toxoid.

Further bacterial antigens may be capsular antigens, saccharide antigens or protein antigens of any of the above. Further bacterial antigens may also include an outer membrane vesicle (OMV) preparation. Additionally, antigens include live, attenuated, and/or purified versions of any of the aforementioned bacteria. The antigens used in the present invention may be derived from gram-negative and/or gram-positive bacteria. The antigens used in the present invention may be derived from aerobic and/or anaerobic bacteria.

### B. Viral Antigens

Viral antigens suitable for use in the invention include inactivated (or killed) virus, attenuated virus, split virus formulations, purified subunit formulations, viral proteins which may be isolated, purified or derived from a virus, and Virus Like Particles (VLPs). Viral antigens may be derived from viruses propagated on cell culture or other substrate. Alternatively, viral antigens may be expressed recombinantly. Viral antigens preferably include epitopes which are exposed on the surface of the virus during at least one stage of its life cycle. Viral antigens are preferably conserved across multiple serotypes or isolates. Viral antigens include antigens derived from one or more of the viruses set forth below as well as the specific antigens examples identified below.

*Orthomyxovirus:* Viral antigens may be derived from an Orthomyxovirus, such as Influenza A, B and C. Orthomyxovirus antigens may be selected from one or more of the viral proteins, including hemagglutinin (HA), neuraminidase (NA), nucleoprotein (NP), matrix protein (M1), membrane protein (M2), one or more of the transcriptase components (PB1, PB2 and PA). Preferred antigens include HA and NA.

Influenza antigens may be derived from interpandemic (annual) flu strains. Alternatively influenza antigens may be derived from strains with the potential to cause a pandemic outbreak (*i.e.*, influenza strains with new haemagglutinin compared to the haemagglutinin in currently circulating strains, or influenza strains which are pathogenic in avian subjects and have the potential to be transmitted horizontally in the human population, or influenza strains which are pathogenic to humans).

*Paramyxoviridae* viruses: Viral antigens may be derived from Paramyxoviridae viruses, such as Pneumoviruses (RSV), Paramyxoviruses (PIV) and Morbilliviruses (Measles). [146-148].

*Pneumovirus:* Viral antigens may be derived from a Pneumovirus, such as Respiratory syncytial virus (RSV), Bovine respiratory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS1 and NS2. Preferred Pneumovirus antigens include F, G and M. See, for example, ref. 149. Pneumovirus antigens may also be formulated in or derived from chimeric viruses. For example, chimeric RSV/PIV viruses may comprise components of both RSV and PIV.

*Paramyxovirus:* Viral antigens may be derived from a Paramyxovirus, such as Parainfluenza virus types 1-4 (PIV), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin-Neuraminidase (HN), Fusion proteins F1 and F2, Nucleoprotein (NP), Phosphoprotein (P), Large protein (L), and Matrix protein (M). Preferred Paramyxovirus proteins include HN, F1 and F2. Paramyxovirus antigens may also be formulated in or derived from chimeric viruses. For example, chimeric RSV/PIV viruses may comprise components of both RSV and PIV. Commercially available mumps vaccines include live attenuated mumps virus, in either a monovalent form or in combination with measles and rubella vaccines (MMR).

*Morbillivirus:* Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

*Picornavirus:* Viral antigens may be derived from Picornaviruses, such as Enteroviruses, Rhinoviruses, Heparnavirus, Cardioviruses and Aphthoviruses. Antigens derived from Enteroviruses, such as Poliovirus are preferred. See refs. 150 & 151.

*Enterovirus:* Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus. Enterovirus antigens are preferably selected from one or more of the following Capsid proteins VP1, VP2, VP3 and VP4. Commercially available polio vaccines include Inactivated Polio Vaccine (IPV) and oral poliovirus vaccine (OPV).

*Heparnavirus:* Viral antigens may be derived from an Heparnavirus, such as Hepatitis A virus (HAV). Commercially available HAV vaccines include inactivated HAV vaccine. [152,153].

*Togavirus:* Viral antigens may be derived from a Togavirus, such as a Rubivirus, an Alphavirus, or an Arterivirus. Antigens derived from Rubivirus, such as Rubella virus, are preferred. Togavirus antigens may be selected from E1, E2, E3, C, NSP-1, NSPO-2, NSP-3 or NSP-4. Togavirus antigens are preferably selected from E1, E2 or E3. Commercially available Rubella vaccines include a live cold-adapted virus, typically in combination with mumps and measles vaccines (MMR).

*Flavivirus:* Viral antigens may be derived from a Flavivirus, such as Tick-borne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, NS3, NS4a, NS4b, and NS5. Flavivirus antigens are preferably selected from PrM, M and E. Commercially available TBE vaccine include inactivated virus vaccines.

*Pestivirus:* Viral antigens may be derived from a Pestivirus, such as Bovine viral diarrhea (BVDV), Classical swine fever (CSFV) or Border disease (BDV).

*Hepadnavirus:* Viral antigens may be derived from a Hepadnavirus, such as Hepatitis B virus. Hepadnavirus antigens may be selected from surface antigens (L, M and S), core antigens (HBc, HBe). Commercially available HBV vaccines include subunit vaccines comprising the surface antigen S protein [153,154].

*Hepatitis C virus:* Viral antigens may be derived from a Hepatitis C virus (HCV). HCV antigens may be selected from one or more of E1, E2, E1/E2, NS345 polyprotein, NS 345-core polyprotein, core, and/or peptides from the nonstructural regions [155,156].

*Rhabdovirus:* Viral antigens may be derived from a Rhabdovirus, such as a Lyssavirus (Rabies virus) and Vesiculovirus (VSV). Rhabdovirus antigens may be selected from glycoprotein (G), nucleoprotein (N), large protein (L), nonstructural proteins (NS). Commercially available Rabies virus vaccine comprises killed virus grown on human diploid cells or fetal rhesus lung cells [157,158].

*Caliciviridae:* Viral antigens may be derived from Calciviridae, such as Norwalk virus, and Norwalk-like Viruses, such as Hawaii Virus and Snow Mountain Virus.

*Coronavirus:* Viral antigens may be derived from a Coronavirus, SARS, Human respiratory coronavirus, Avian infectious bronchitis (IBV), Mouse hepatitis virus (MHV), and Porcine transmissible gastroenteritis virus (TGEV). Coronavirus antigens may be selected from spike (S), envelope (E), matrix (M), nucleocapsid (N), and/or Hemagglutinin-esterase glycoprotein (HE). Preferably, the Coronavirus antigen is derived from a SARS virus. SARS viral antigens are described in ref. 159.

*Retrovirus:* Viral antigens may be derived from a Retrovirus, such as an Oncovirus, a Lentivirus or a Spumavirus. Oncovirus antigens may be derived from HTLV-1, HTLV-2 or HTLV-5. Lentivirus antigens may be derived from HIV-1 or HIV-2. Retrovirus antigens may be selected from gag, pol, env, tax, tat, rex, rev, nef, vif, vpu, and vpr. HIV antigens may be selected from gag (p24gag and p55gag), env (gp160, gp120 and gp41), pol, tat, nef, rev vpu, miniproteins, (preferably p55 gag and gp140v delete). HIV antigens may be derived from one or more of the following strains: HIV_{IIIb}, HIV_{SF2}, HIV_{LAV}, HIV_{LAI,} HIV_{MN}, HIV-1_{CM235}, HIV-1_{US4}.

*Reovirus:* Viral antigens may be derived from a Reovirus, such as an Orthoreovirus, a Rotavirus, an Orbivirus, or a Coltivirus. Reovirus antigens may be selected from structural proteins λ1, λ2, λ3, µ1, µ2, σ1, σ2, or σ3, or nonstructural proteins σNS, µNS, or σ1s. Preferred Reovirus antigens may be derived from a Rotavirus. Rotavirus antigens may be selected from VP1, VP2, VP3, VP4 (or the cleaved product VP5 and VP8), NSP 1, VP6, NSP3, NSP2, VP7, NSP4, and/or NSP5. Preferred Rotavirus antigens include VP4 (or the cleaved product VP5 and VP8), and VP7.

*Parvovirus:* Viral antigens may be derived from a Parvovirus, such as Parvovirus B19. Parvovirus antigens may be selected from VP-1, VP-2, VP-3, NS-1 and/or NS-2. Preferably, the Parvovirus antigen is capsid protein VP-2.

*Delta hepatitis virus (HDV):* Viral antigens may be derived HDV, particularly δ-antigen from HDV (see, e.g., ref. 160).

*Hepatitis E virus (HEV):* Viral antigens may be derived from HEV.

*Hepatitis G virus (HGV):* Viral antigens may be derived from HGV.

*Human Herpesvirus:* Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HPV6), Human Herpesvirus 7 (HHV7), and Human Herpesvirus 8 (HHV8). Human Herpesvirus antigens may be selected from immediate early proteins (α), early proteins (β), and late proteins (γ). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigens may be selected from core, nucleocapsid, tegument, or envelope proteins. A live attenuated VZV vaccine is commercially available. EBV antigens may be selected from early antigen (EA) proteins, viral capsid antigen (VCA), and glycoproteins of the membrane antigen (MA). CMV antigens may be selected from capsid proteins, envelope glycoproteins (such as gB and gH), and tegument proteins

*Papovaviruses:* Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (L1) and (L2), or E1 - E7, or fusions thereof. HPV antigens are preferably formulated into virus-like particles (VLPs). Polyomyavirus viruses include BK virus and JK virus. Polyomavirus antigens may be selected from VP 1, VP2 or VP3.

### C. Fungal Antigens

Fungal antigens may be derived from one or more of the fungi set forth below.

Fungal antigens may be derived from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme.*

Fungal pathogens include *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei,* Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Processes for producing a fungal antigens are well known in the art [161]. In a preferred method a solubilized fraction extracted and separated from an insoluble fraction obtainable from fungal cells of which cell wall has been substantially removed or at least partially removed, characterized in that the process comprises the steps of: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

### D. STD Antigens

The compositions of the invention may include one or more antigens derived from a sexually transmitted disease (STD). Such antigens may provide for prophylactis or therapy for STD's such as chlamydia, genital herpes, hepatits (such as HCV), genital warts, gonorrhoea, syphilis and/or chancroid [162]. Antigens may be derived from one or more viral or bacterial STD's. Viral STD antigens for use in the invention may be derived from, for example, HIV, herpes simplex virus (HSV-1 and HSV-2), human papillomavirus (HPV), and hepatitis (HCV). Bacterial STD antigens for use in the invention may be derived from, for example, *Neisseria gonorrhoeae, Chlamydia trachomatis, Treponema pallidum, Haemophilus ducreyi, Escherichia coli,* and *Streptococcus agalactiae.* Examples of specific antigens derived from these pathogens are described above.

### E. Respiratory Antigens

The compositions of the invention may include one or more antigens derived from a pathogen which causes respiratory disease. For example, respiratory antigens may be derived from a respiratory virus such as Orthomyxoviruses (influenza), Pneumovirus (RSV), Paramyxovirus (PIV), Morbillivirus (measles), Togavirus (Rubella), VZV, and Coronavirus (SARS). Respiratory antigens may be derived from a bacteria which causes respiratory disease, such as *Streptococcus pneumoniae, Pseudomonas aeruginosa, Bordetella pertussis, Mycobacterium tuberculosis, Mycoplasma pneumoniae, Chlamydia pneumoniae, Bacillus anthracis,* and *Moraxella catarrhalis.* Examples of specific antigens derived from these pathogens are described above.

### F. Pediatric Vaccine Antigens

The compositions of the invention may include one or more antigens suitable for use in pediatric subjects. Pediatric subjects are typically less than about 3 years old, or less than about 2 years old, or less than about 1 years old. Pediatric antigens may be administered multiple times over the course of 6 months, 1, 2 or 3 years. Pediatric antigens may be derived from a virus which may target pediatric populations and/or a virus from which pediatric populations are susceptible to infection. Pediatric viral antigens include antigens derived from one or more of Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), and Varicella-zoster virus (VZV), Epstein Barr virus (EBV). Pediatric bacterial antigens include antigens derived from one or more of *Streptococcus pneumoniae, Neisseria meningitidis, Streptococcus pyogenes* (Group A Streptococcus), *Moraxella catarrhalis, Bordetella pertussis, Staphylococcus aureus, Clostridium tetani* (Tetanus), *Corynebacterium diphtheriae* (Diphtheria), *Haemophilus influenzae* type B (Hib), *Pseudomonas aeruginosa, Streptococcus agalactiae* (Group B Streptococcus), and *Escherichia coli.* Examples of specific antigens derived from these pathogens are described above.

### G. Antigens suitable for use in Elderly or Immunocompromised Individuals

The compositions of the invention may include one or more antigens suitable for use in elderly or immunocompromised individuals. Such individuals may need to be vaccinated more frequently, with higher doses or with adjuvanted formulations to improve their immune response to the targeted antigens. Antigens which may be targeted for use in Elderly or Immunocompromised individuals include antigens derived from one or more of the following pathogens: *Neisseria meningitidis, Streptococcus pneumoniae, Streptococcus pyogenes* (Group A Streptococcus), *Moraxella catarrhalis, Bordetella pertussis, Staphylococcus aureus, Staphylococcus epidermis, Clostridium tetani* (Tetanus), *Cornynebacterium diphtheriae* (Diphtheria), *Haemophilus influenzae* type B (Hib), *Pseudomonas aeruginosa, Legionella pneumophila, Streptococcus agalactiae* (Group B Streptococcus), *Enterococcus faecalis, Helicobacter pylori, Chlamydia pneumoniae,* Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), Varicella-zoster virus (VZV), Epstein Barr virus (EBV), Cytomegalovirus (CMV). Examples of specific antigens derived from these pathogens are described above.

### H. Antigens suitable for use in Adolescent Vaccines

The compositions of the invention may include one or more antigens suitable for use in adolescent subjects. Adolescents may be in need of a boost of a previously administered pediatric antigen. Pediatric antigens which may be suitable for use in adolescents are described above. In addition, adolescents may be targeted to receive antigens derived from an STD pathogen in order to ensure protective or therapeutic immunity before the beginning of sexual activity. STD antigens which may be suitable for use in adolescents are described above.

### I. Tumor Antigens

One embodiment of the invention involves a tumor antigen or cancer antigen. Tumor antigens can be, for example, peptide-containing tumor antigens, such as a polypeptide tumor antigen or glycoprotein tumor antigens. A tumor antigen can also be, for example, a saccharide-containing tumor antigen, such as a glycolipid tumor antigen or a ganglioside tumor antigen. The tumor antigen can further be, for example, a polynucleotide-containing tumor antigen that expresses a polypeptide-containing tumor antigen, for instance, an RNA vector construct or a DNA vector construct, such as plasmid DNA.

Tumor antigens appropriate for the practice of the present invention encompass a wide variety of molecules, such as (a) polypeptide-containing tumor antigens, including polypeptides (which can range, for example, from 8-20 amino acids in length, although lengths outside this range are also common), lipopolypeptides and glycoproteins, (b) saccharide-containing tumor antigens, including poly-saccharides, mucins, gangliosides, glycolipids and glycoproteins, and (c) polynucleotides that express antigenic polypeptides.

The tumor antigens can be, for example, (a) full length molecules associated with cancer cells, (b) homologs and modified forms of the same, including molecules with deleted, added and/or substituted portions, and (c) fragments of the same. Tumor antigens can be provided in recombinant form. Tumor antigens include, for example, class I-restricted antigens recognized by CD8+ lymphocytes or class II-restricted antigens recognized by CD4+ lymphocytes.

Numerous tumor antigens are known in the art, including: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors), (b) mutated antigens, for example, p53 (associated with various solid tumors, e.g., colorectal, lung, head and neck cancer), p21/Ras (associated with, e.g., melanoma, pancreatic cancer and colorectal cancer), CDK4 (associated with, e.g., melanoma), MUM1 (associated with, e.g., melanoma), caspase-8 (associated with, e.g., head and neck cancer), CIA 0205 (associated with, e.g., bladder cancer), HLA-A2-R1701, beta catenin (associated with, e.g., melanoma), TCR (associated with, e.g., T-cell non-Hodgkins lymphoma), BCR-abl (associated with, e.g., chronic myelogenous leukemia), triosephosphate isomerase, KIA 0205, CDC-27, and LDLR-FUT, (c) over-expressed antigens, for example, Galectin 4 (associated with, e.g., colorectal cancer), Galectin 9 (associated with, e.g., Hodgkin's disease), proteinase 3 (associated with, e.g., chronic myelogenous leukemia), WT 1 (associated with, e.g., various leukemias), carbonic anhydrase (associated with, e.g., renal cancer), aldolase A (associated with, e.g., lung cancer), PRAME (associated with, e.g., melanoma), HER-2/neu (associated with, e.g., breast, colon, lung and ovarian cancer), alpha-fetoprotein (associated with, e.g., hepatoma), KSA (associated with, e.g., colorectal cancer), gastrin (associated with, e.g., pancreatic and gastric cancer), telomerase catalytic protein, MUC-1 (associated with, e.g., breast and ovarian cancer), G-250 (associated with, e.g., renal cell carcinoma), p53 (associated with, e.g., breast, colon cancer), and carcinoembryonic antigen (associated with, e.g., breast cancer, lung cancer, and cancers of the gastrointestinal tract such as colorectal cancer), (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, e.g., melanoma), (e) prostate associated antigens such as PAP, PSA, PSMA, PSH-P1, PSM-P1, PSM-P2, associated with e.g., prostate cancer, (f) immunoglobulin idiotypes (associated with myeloma and B cell lymphomas, for example), and (g) other tumor antigens, such as polypeptide- and saccharide-containing antigens including (i) glycoproteins such as sialyl Tn and sialyl Le^{x} (associated with, e.g., breast and colorectal cancer) as well as various mucins; glycoproteins may be coupled to a carrier protein (e.g., MUC-1 may be coupled to KLH); (ii) lipopolypeptides (e.g., MUC-1 linked to a lipid moiety); (iii) polysaccharides (e.g., Globo H synthetic hexasaccharide), which may be coupled to a carrier proteins (e.g., to KLH), (iv) gangliosides such as GM2, GM12, GD2, GD3 (associated with, e.g., brain, lung cancer, melanoma), which also may be coupled to carrier proteins (e.g., KLH).

Additional tumor antigens which are known in the art include p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like. These as well as other cellular components are described for example in reference 163 and references cited therein.

Polynucleotide-containing antigens in accordance with the present invention typically comprise polynucleotides that encode polypeptide cancer antigens such as those listed above. Preferred polynucleotide-containing antigens include DNA or RNA vector constructs, such as plasmid vectors (e.g., pCMV), which are capable of expressing polypeptide cancer antigens *in vivo.* Tumor antigens may be derived, for example, from mutated or altered cellular components. After alteration, the cellular components no longer perform their regulatory functions, and hence the cell may experience uncontrolled growth. Representative examples of altered cellular components include ras, p53, Rb, altered protein encoded by the Wilms' tumor gene, ubiquitin, mucin, protein encoded by the DCC, APC, and MCC genes, as well as receptors or receptor-like structures such as neu, thyroid hormone receptor, platelet derived growth factor (PDGF) receptor, insulin receptor, epidermal growth factor (EGF) receptor, and the colony stimulating factor (CSF) receptor. These as well as other cellular components are described for example in ref. 164 and references cited therein.

Additionally, bacterial and viral antigens, may be used in conjunction with the compositions of the present invention for the treatment of cancer. In particular, carrier proteins, such as CRM197, tetanus toxoid, or *Salmonella typhimurium* antigen can be used in conjunction/conjugation with compounds of the present invention for treatment of cancer. The cancer antigen combination therapies will show increased efficacy and bioavailability as compared with existing therapies.

Additional information on cancer or tumor antigens can be found, for example, in reference 165 (*e.g*. Tables 3 & 4), in reference 166 (*e.g.* Table 1) and in references 167 to 189.

Immunisation can also be used against Alzheimer's disease *e.g*. using Abeta as an antigen [190].

### J. Antigen Formulations

In other aspects of the invention, methods of producing microparticles having adsorbed antigens are provided. The methods comprise: (a) providing an emulsion by dispersing a mixture comprising (i) water, (ii) a detergent, (iii) an organic solvent, and (iv) a biodegradable polymer selected from the group consisting of a poly(α-hydroxy acid), a polyhydroxy butyric acid, a polycaprolactone, a polyorthoester, a polyanhydride, and a polycyanoacrylate. The polymer is typically present in the mixture at a concentration of about 1% to about 30% relative to the organic solvent, while the detergent is typically present in the mixture at a weight-to-weight detergent-to-polymer ratio of from about 0.00001:1 to about 0.1:1 (more typically about 0.0001:1 to about 0.1:1, about 0.001:1 to about 0.1:1, or about 0.005:1 to about 0.1:1); (b) removing the organic solvent from the emulsion; and (c) adsorbing an antigen on the surface of the microparticles. In certain embodiments, the biodegradable polymer is present at a concentration of about 3% to about 10% relative to the organic solvent.

Microparticles for use herein will be formed from materials that are sterilizable, non-toxic and biodegradable. Such materials include, without limitation, poly(α-hydroxy acid), polyhydroxybutyric acid, polycaprolactone, polyorthoester, polyanhydride, PACA, and polycyanoacrylate. Preferably, microparticles for use with the present invention are derived from a poly(α-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered macromolecule. These parameters are discussed more fully below.

Additional formulation methods and antigens (especially tumor antigens) are provided in ref. 191.

### Medical methods and uses

Once formulated, the compositions of the invention can be administered directly to the subject. The subjects to be treated can be animals; in particular, human subjects can be treated. The compositions may be formulated as vaccines that are particularly useful for vaccinating children and teenagers. They may be delivered by systemic and/or mucosal routes.

Typically, the compositions are prepared as injectables, either as liquid solutions or suspensions; solid forms suitable for solution in, or suspension in, liquid vehicles prior to injection may also be prepared. Direct delivery of the compositions will generally be parenteral (*e.g*. by injection, either subcutaneously, intraperitoneally, intravenously or intramuscularly or delivered to the interstitial space of a tissue). The compositions can also be administered into a lesion. Other modes of administration include oral and pulmonary administration, suppositories, and transdermal or transcutaneous applications (*e.g*. see ref. 192), needles, and hyposprays. Dosage treatment may be a single dose schedule or a multiple dose schedule (*e.g*. including booster doses).

Vaccines of the invention are preferably sterile. They are preferably pyrogen-free. They are preferably buffered *e.g*. at between pH 6 and pH 8, generally around pH 7. Where a vaccine comprises an aluminium hydroxide salt, it is preferred to use a histidine buffer [193].

Vaccines of the invention may comprise detergent (*e.g*. a Tween, such as Tween 80) at low levels *(*e.g*.* <0.01%). Vaccines of the invention may comprise a sugar alcohol (*e.g*. mannitol) or trehalose *e.g*. at around 15mg/ml, particularly if they are to be lyophilised.

Optimum doses of individual antigens can be assessed empirically. In general, however, saccharide antigens of the invention will be administered at a dose of between 0.1 and 100µg of each saccharide per dose, with a typical dosage volume of 0.5ml. The dose is typically between 5 and 20µg per saccharide per dose. These values are measured as saccharide.

Vaccines according to the invention may either be prophylactic (*i.e*. to prevent infection) or therapeutic (*i.e.* to treat disease after infection), but will typically be prophylactic.

The invention provides a conjugate of the invention for use in medicine.

The invention is useful in a method of raising an immune response in a patient, comprising administering to a patient a conjugate according to the invention. The immune response is preferably protective against meningococcal disease, and may comprise a humoral immune response and/or a cellular immune response. The patient is preferably a child. The method may raise a booster response, in a patient that has already been primed against meningococcus.

The medicament is preferably an immunogenic composition (*e.g*. a vaccine). The medicament is preferably for the prevention and/or treatment of a disease caused by a *Neisseria* (*e.g*. meningitis, septicaemia, gonorrhoea *etc*.), The prevention and/or treatment of bacterial meningitis is thus preferred.

Vaccines can be tested in standard animal models (*e.g*. see ref. 194).

A kit can comprise a) a first conjugate of the invention and b) a second conjugate of the invention.

### Adjuvants

Conjugates of the invention may be administered in conjunction with other immunoregulatory agents. In particular, compositions will usually include an adjuvant. Adjuvants which may be used in compositions of the invention include, but are not limited to:

### A. Mineral-containing compositions

Mineral containing compositions suitable for use as adjuvants in the invention include mineral salts, such as aluminium salts and calcium salts. Such mineral compositions may include mineral salts such as hydroxides (*e.g.* oxyhydroxides), phosphates (*e.g.* hydroxyphosphates, orthophosphates), sulphates, *etc.* [*e.g.* see chapters 8 & 9 of ref. 195], or mixtures of different mineral compounds (*e.g*. a mixture of a phosphate and a hydroxide adjuvant, optionally with an excess of the phosphate), with the compounds taking any suitable form (*e.g*. gel, crystalline, amorphous, *etc.*), and with adsorption to the salt(s) being preferred. The mineral containing compositions may also be formulated as a particle of metal salt [196].

Aluminum salts may be included in compositions of the invention such that the dose of Al³⁺ is between 0.2 and 1.0 mg per dose.

A typical aluminium phosphate adjuvant is amorphous aluminium hydroxyphosphate with PO₄/Al molar ratio between 0.84 and 0.92, included at 0.6mg Al³⁺/ml. Adsorption with a low dose of aluminium phosphate may be used *e.g*. between 50 and 100µg Al³⁺ per conjugate per dose. Where an aluminium phosphate it used and it is desired not to adsorb an antigen to the adjuvant, this is favoured by including free phosphate ions in solution (*e.g*. by the use of a phosphate buffer).

### B. Oil Emulsions

Oil emulsion compositions suitable for use as adjuvants with conjugates of the invention include squalene-water emulsions, such as MF59 (5% Squalene, 0.5% Tween 80, and 0.5% Span 85, formulated into submicron particles using a microfluidizer) [Chapter 10 of ref. 195; see also refs. 197-199]. MF59 is used as the adjuvant in the FLUAD™ influenza virus trivalent subunit vaccine. The MF59 emulsion advantageously includes citrate ions *e.g*. 10mM sodium citrate buffer.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80 (polyoxyelthylenesorbitan monooleate), and/or 0.25-1.0% Span 85 (sorbitan trioleate), and, optionally, N-acetylmuramyl-L-alanyl-D-isogluatminyl-L-alanine-2-(1'-2'-dipalmitoyl-sn-glycero-3-hydroxyphosphophoryloxy)-ethylamine (MTP-PE). Submicron oil-in-water emulsions, methods of making the same and immunostimulating agents, such as muramyl peptides, for use in the compositions, are described in detail in references 197 & 200-201.

An emulsion of squalene, a tocopherol, and Tween 80 can be used. The emulsion may include phosphate buffered saline. It may also include Span 85 (*e.g*. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.

An emulsion of squalene, a tocopherol, and a Triton detergent (*e.g*. Triton X-100) can be used.

An emulsion of squalane, polysorbate 80 and poloxamer 401 ("Pluronic™ L121") can be used. The emulsion can be formulated in phosphate buffered saline, pH 7.4. This emulsion is a useful delivery vehicle for muramyl dipeptides, and has been used with threonyl-MDP in the "SAF-1" adjuvant [202] (0.05-1% Thr-MDP, 5% squalane, 2.5% Pluronic L121 and 0.2% polysorbate 80). It can also be used without the Thr-MDP, as in the "AF" adjuvant [203] (5% squalane, 1.25% Pluronic L121 and 0.2% polysorbate 80). Microfluidisation is preferred.

Complete Freund's adjuvant (CFA) and incomplete Freund's adjuvant (IFA) may also be used as adjuvants.

### C. Saponin formulations [chapter 22 of ref. 195]

Saponin formulations may also be used as adjuvants of conjugates of the invention. Saponins are a heterologous group of sterol glycosides and triterpenoid glycosides that are found in the bark, leaves, stems, roots and even flowers of a wide range of plant species. Saponins isolated from the bark of the *Quillaia saponaria* Molina tree have been widely studied as adjuvants. Saponin can also be commercially obtained from *Smilax ornata* (sarsaparilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon^{™}.

Saponin compositions have been purified using HPLC and RP-HPLC. Specific purified fractions using these techniques have been identified, including QS7, QS 17, QS 18, QS21, QH-A, QH-B and QH-C. Preferably, the saponin is QS21. A method of production of QS21 is disclosed in ref. 204. Saponin formulations may also comprise a sterol, such as cholesterol [205].

Combinations of saponins and cholesterols can be used to form unique particles called immunostimulating complexs (ISCOMs) [chapter 23 of ref. 195]. ISCOMs typically also include a phospholipid such as phosphatidylethanolamine or phosphatidylcholine. Any known saponin can be used in ISCOMs. Preferably, the ISCOM includes one or more of QuilA, QHA and QHC. ISCOMs are further described in refs. 205-207. Optionally, the ISCOMS may be devoid of additional detergent(s) [208].

A review of the development of saponin based adjuvants can be found in refs. 209 & 210.

### D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in refs. 211-216. Virosomes are discussed further in, for example, ref. 217

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 218. Such "small particles" of 3dMPL are small enough to be sterile filtered through a 0.22µm membrane [218]. Other non-toxic LPS derivatives include monophosphoryl lipid A mimics, such as aminoalkyl glucosaminide phosphate derivatives *e.g*. RC-529 [219,220].

Lipid A derivatives include derivatives of lipid A from *Escherichia coli* such as OM-174. OM-174 is described for example in refs. 221 & 222.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 223, 224 and 225 disclose possible analog substitutions *e.g*. replacement of guanosine with 2'-deoxy-7-deazaguanosine. The adjuvant effect of CpG oligonucleotides is further discussed in refs. 226-231.

The CpG sequence may be directed to TLR9, such as the motif GTCGTT or TTCGTT [232]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 233-235. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 232 & 236-238.

Bacterial ADP-ribosylating toxins and detoxified derivatives thereof may be used as adjuvants in the invention. Preferably, the protein is derived from *E.coli* (*E.coli* heat labile enterotoxin "LT"), cholera ("CT"), or pertussis ("PT"). The use of detoxified ADP-ribosylating toxins as mucosal adjuvants is described in ref. 239 and as parenteral adjuvants in ref. 240. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivaties thereof, particularly LT-K63 and LT-R72, as adjuvants can be found in refs. 241-248. Numerical reference for amino acid substitutions is preferably based on the alignments of the A and B subunits of ADP-ribosylating toxins set forth in ref. 249, specifically incorporated herein by reference in its entirety.

Compounds of formula I, II or III, or salts thereof, can also be used as adjuvants: 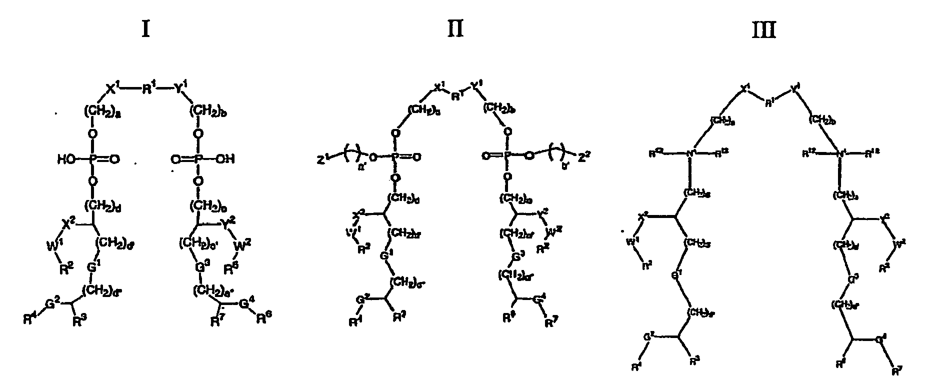 as defined in reference 250, such as 'ER 803058', 'ER 803732', 'ER 804053', ER 804058', 'ER 804059', 'ER 804442', 'ER 804680', 'ER 804764', ER 803022 or 'ER 804057' *e.g*.:

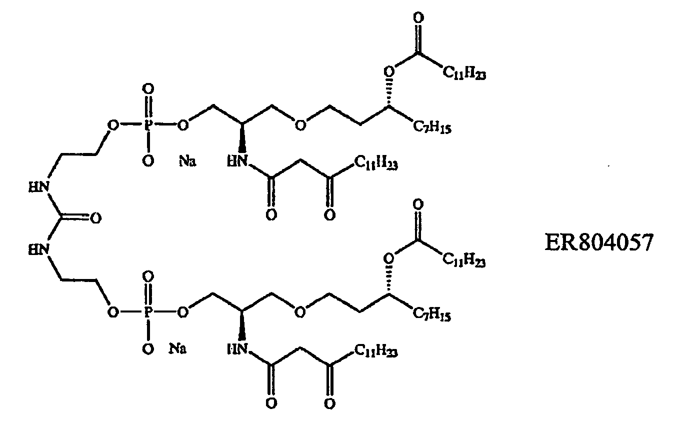

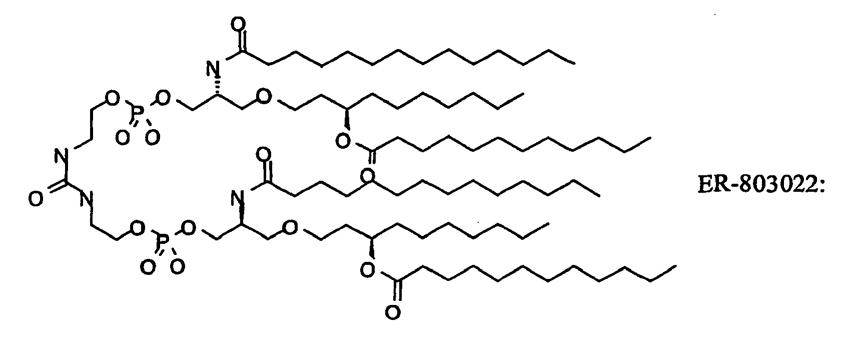

### F. Human immunomodulators

Human immunomodulators suitable for use as adjuvants in the invention include cytokines, such as interleukins *(e.g.* IL-1, IL-2, IL-4, IL-5, IL-6, IL-7, IL-12 [251], IL-23, IL-27 [252] *etc*.) [253], interferons (*e.g*. interferon-γ), macrophage colony stimulating factor, tumor necrosis factor and macrophage inflammatory protein-1alpha (MIP-1alpha) and MIP-1beta [254].

### G. Bioadhesives and Mucoadhesives

Bioadhesives and mucoadhesives may also be used as adjuvants in the invention. Suitable bioadhesives include esterified hyaluronic acid microspheres [255] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [256].

### H. Microparticles

Microparticles may also be used as adjuvants in the invention. Microparticles (*i.e*. a particle of ~100nm to ~150µm in diameter, more preferably ~200nm to ~30µm in diameter, and most preferably ~500nm to ~10µm in diameter) formed from materials that are biodegradable and non-toxic (*e.g*. a poly(α-hydroxy acid), a polyhydroxybutyric acid, a polyorthoester, a polyanhydride, a polycaprolactone, *etc*.)*,* with poly(lactide-co-glycolide) are preferred, optionally treated to have a negatively-charged surface (*e.g*. with SDS) or a positively-charged surface (*e.g*. with a cationic detergent, such as CTAB).

### I. Liposomes (Chapters 13 & 14 of ref. 195)

Examples of liposome formulations suitable for use as adjuvants are described in refs. 257-259.

### J. Polyoxyethylene ether and polyoxyethylene ester formulations

Adjuvants suitable for use in the invention include polyoxyethylene ethers and polyoxyethylene esters [260]. Such formulations further include polyoxyethylene sorbitan ester surfactants in combination with an octoxynol [261] as well as polyoxyethylene alkyl ethers or ester surfactants in combination with at least one additional non-ionic surfactant such as an octoxynol [262]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### K. Polyphosphazene (PCPP)

PCPP (poly[di(carboxylatophenoxy)phosphazene]) formulations are described, for example, in refs. 263 and 264.

### L. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn-*glycero-3-hydroxyphosphoryloxy)-ethylamine MTP-PE).

### M. Imidazoquinolone Compounds.

Examples of imidazoquinolone compounds suitable for use as adjuvants in the invention include Imiquamod and its homologues (*e.g*. "Resiquimod 3M"), described further in refs. 265 and 266.

### N. Thiosemicarbazone Compounds.

Examples of thiosemicarbazone compounds, as well as methods of formulating, manufacturing, and screening for compounds all suitable for use as adjuvants in the invention include those described in ref. 267. The thiosemicarbazones are particularly effective in the stimulation of human peripheral blood mononuclear cells for the production of cytokines, such as TNF-α.

### O. Tryptanthrin Compounds.

Examples of tryptanthrin compounds, as well as methods of formulating, manufacturing, and screening for compounds all suitable for use as adjuvants in the invention include those described in ref. 268. The tryptanthrin compounds are particularly effective in the stimulation of human peripheral blood mononuclear cells for the production of cytokines, such as TNF-α.

### P. Nucleoside analogs

Various nucleoside analogs can be used as adjuvants, such as (a) Isatorabine (ANA-245; 7-thia-8-oxoguanosine): 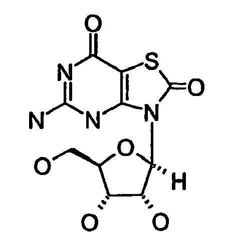 and prodrugs thereof; (b) ANA975; (c) ANA-025-1; (d) ANA380; (e) the compounds disclosed in references 269 to 271; (f) a compound having the formula: 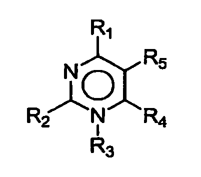 wherein:
R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 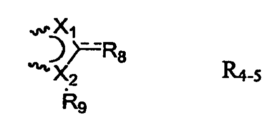 the binding being achieved at the bonds indicated by a 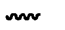
X₁ and X₂ are each independently N, C, O, or S;
R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚR_{c}, or -C(O)-R_{d};
R₉ is H, C₁₋₆ alkyl, substituted C₁₋₆ alkyl, heterocyclyl, substituted heterocyclyl or R₉ₐ, wherein R₉ₐ is: 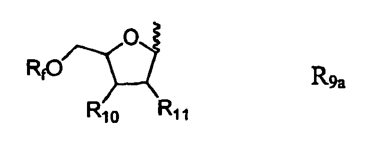 the binding being achieved at the bond indicated by a 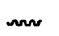
R₁₀ and R₁₁ are each independently H, halo, C₁₋₆ alkoxy, substituted C₁₋₆ alkoxy, -NRₐR_{b}, or -OH;
each Rₐ and R_{b} is independently H, C₁₋₆ alkyl, substituted C₁₋₆ alkyl, -C(O)R_{d}, C₆₋₁₀ aryl;
each R_{c} is independently H, phosphate, diphosphate, triphosphate, C₁₋₆ alkyl, or substituted C₁₋₆ alkyl;
each R_{d} is independently H, halo, C₁₋₆ alkyl, substituted C₁₋₆ alkyl, C₁₋₆ alkoxy, substituted C₁₋₆ alkoxy, NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
each R_{f} is independently H, C₁₋₆ alkyl, substituted C₁₋₆ alkyl, -C(O)R_{d}, phosphate, diphosphate, or triphosphate;
each n is independently 0, 1, 2, or 3;
each p is independently 0, 1, or 2; or
or (g) a pharmaceutically acceptable salt of any of (a) to (f), a tautomer of any of (a) to (f), or a pharmaceutically acceptable salt of the tautomer.

### Q. Lipids linked to a phosphate-containing acyclic backbone

Adjuvants containing lipids linked to a phosphate-containing acyclic backbone include the TLR4 antagonist E5564 [272,273]:

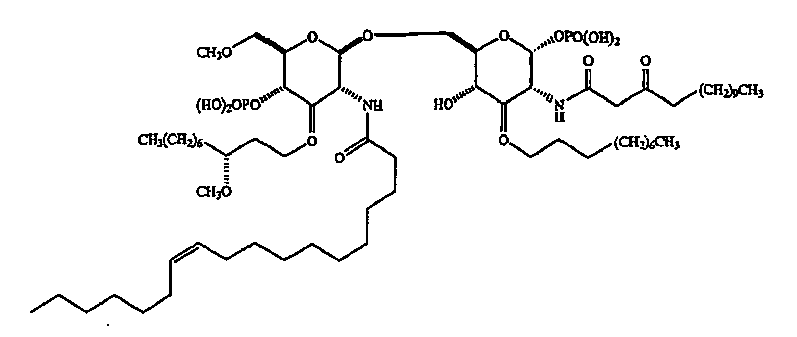

### R. Small molecule immunopotentiators (SMIPs)

SMIPs include:
- N2-methyl-1-(2-methylpropyl)-1H-imidazo[4,5-c]quinoline-2,4-diamine;
- N2,N2-dimethyl-1-(2-methylpropyl)-1H-imidazo[4,5-c]quinoline-2,4-diamine;
- N2-ethyl-N2-methyl-1-(2-methylpropyl)-1H-imidazo[4,5-c]quinoline-2,4-diamine;
- N2-methyl-1-(2-methylpropyl)-N2-propyl-1H-imidazo[4,5-c]quinoline-2,4-diamine;
- 1-(2-methylpropyl)-N2-propyl-1H-imidazo[4,5-c]quinoline-2,4-diamine;
- N2-butyl-1-(2-methylpropyl)-1H-imidazo[4,5-c]quinoline-2,4-diamine;
- N2-butyl-N2-methyl-1-(2-methylpropyl)-1H-imidazo[4,5-c]quinoline-2,4-diamine;
- N2-methyl-1-(2-methylpropyl)-N2-pentyl-1H-imidazo[4,5-c]quinoline-2,4-diamine;
- N2-methyl-1-(2-methylpropyl)-N2-prop-2-enyl-1H-imidazo[4,5-c]quinoline-2,4-diamine;
- 1-(2-methylpropyl)-2-[(phenylmethyl)thio]-1H-imidazo[4,5-c]quinolin-4-amine;
- 1-(2-methylpropyl)-2-(propylthio)-1H-imidazo[4,5-c]quinolin-4-amine ;
- 2-[[4-amino-1-(2-methylpropyl)-1H-imidazo[4,5-c]quinolin-2-yl](methyl)amino]ethanol;
- 2-[[4-amino-1-(2-methylpropyl)-1H-imidazo[4,5-c]quinolin-2-yl](methyl)amino]ethyl acetate;
- 4-amino-1-(2-methylpropyl)-1,3-dihydro-2H-imidazo[4,5-c]quinolin-2-one;
- N2-butyl-1-(2-methylpropyl)-N4,N4-bis(phenylmethyl)-1H-imidazo[4,5-c]quinoline-2,4-diamine;
- N2-butyl-N2-methyl-1-(2-methylpropyl)-N4,N4-bis(phenylmethyl)-1H-imidazo[4,5-c]quinoline-2,4-diamine;
- N2-methyl-1-(2-methylpropyl)-N4,N4-bis(phenylmethyl)-1H-imidazo[4,5-c]quinoline-2,4-diamine;
- N2,N2-dimethyl-1-(2-methylpropyl)-N4,N4-bis(phenylmethyl)-1H-imidazo[4,5-c]quinoline-2,4-diamine;
- 1-(4-amino-2-[methyl(propyl)amino]-1H-imidazo[4,5-c]quinolin-1-yl}-2-methylpropan-2-ol;
- 1-[4-amino-2-(propylamino)-1H-imidazo[4,5-c]quinolin-1-yl]-2-methylpropan-2-ol;
- N4,N4-dibenzyl-1-(2-methoxy-2-methylpropyl)-N2-propyl-1H-imidazo[4,5-c]quinoline-2,4-diamine.

### S. Proteosomes

One adjuvant is an outer membrane protein proteosome preparation prepared from a first Gram-negative bacterium in combination with a liposaccharide preparation derived from a second Gram-negative bacterium, wherein the outer membrane protein proteosome and liposaccharide preparations form a stable non-covalent adjuvant complex. Such complexes include "IVX-908", a complex comprised of *Neisseria meningitidis* outer membrane and lipopolysaccharides. They have been used as adjuvants for influenza vaccines [274].

### T. Other adjuvants

Other substances that act as immunostimulating agents are disclosed in references 195 and 275. Further useful adjuvant substances include:
- Methyl inosine 5'-monophosphate ("MIMP") [276].
- A polyhydroxlated pyrrolizidine compound [277], such as one having formula: 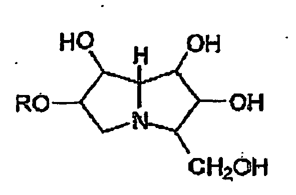 where R is selected from the group comprising hydrogen, straight or branched, unsubstituted or substituted, saturated or unsaturated acyl, alkyl (*e.g*. cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-epi-casuarine, 3,7-diepi-casuarine, *etc*.
- A gamma inulin [278] or derivative thereof, such as algammulin.
- Compounds disclosed in reference 279.
- Compounds disclosed in reference 280, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [281,282], Hydrapthalamide compounds, Benzophenone compounds, Isoxazole compounds, Sterol compounds, Quinazilinone compounds, Pyrrole compounds [283], Anthraquinone compounds, Quinoxaline compounds, Triazine compounds, Pyrazalopyrimidine compounds, and Benzazole compounds [284].
- Loxoribine (7-allyl-8-oxoguanosine) [285].

A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [286].

The invention may also comprise combinations of aspects of one or more of the adjuvants identified above. For example, the following combinations may be used as adjuvant compositions in the invention: (1) a saponin and an oil-in-water emulsion [287]; (2) a saponin (*e.g*. QS21) + a non-toxic LPS derivative (*e.g*. 3dMPL) [288]; (3) a saponin (*e.g*. QS21) + a non-toxic LPS derivative (*e.g*. 3dMPL) + a cholesterol; (4) a saponin (*e.g*. QS21) + 3dMPL + IL-12 (optionally + a sterol) [289]; (5) combinations of 3dMPL with, for example, QS21 and/or oil-in-water emulsions [290]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi^{™} adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™; (8) one or more mineral salts (such as an aluminum salt) + a non-toxic derivative of LPS (such as 3dMPL); and (9) one or more mineral salts (such as an aluminum salt) + an immunostimulatory oligonucleotide (such as a nucleotide sequence including a CpG motif).

### Definitions

The term "comprising" encompasses "including" as well as "consisting" *e.g*. a composition "comprising" X may consist exclusively of X or may include something additional *e.g*. X + Y.

The term "about" in relation to a numerical value x means, for example, *x*±10%. All numerical values herein can be considered to be qualified by "about", unless the context indicates otherwise.

The word "substantially" does not exclude "completely" *e.g*. a composition which is "substantially free" from Y may be completely free from Y. Where necessary, the word "substantially" may be omitted from the definition of the invention.

### BRIEF DESCRIPTION OF DRAWINGS

Figure 1 shows the various possible combinations of carrier and saccharide antigen (A) two monovalent conugates, (B) a monovalent conjugate demonstrating that each carrier protein molecule may be bound to more than one saccharide antigen molecule and (C) a multivalent conjugate where more than one antigenically distinct saccharide is attached to each carrier protein molecule.

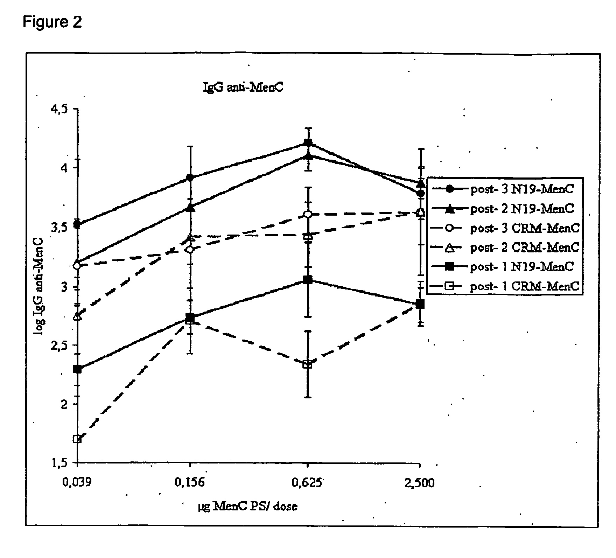

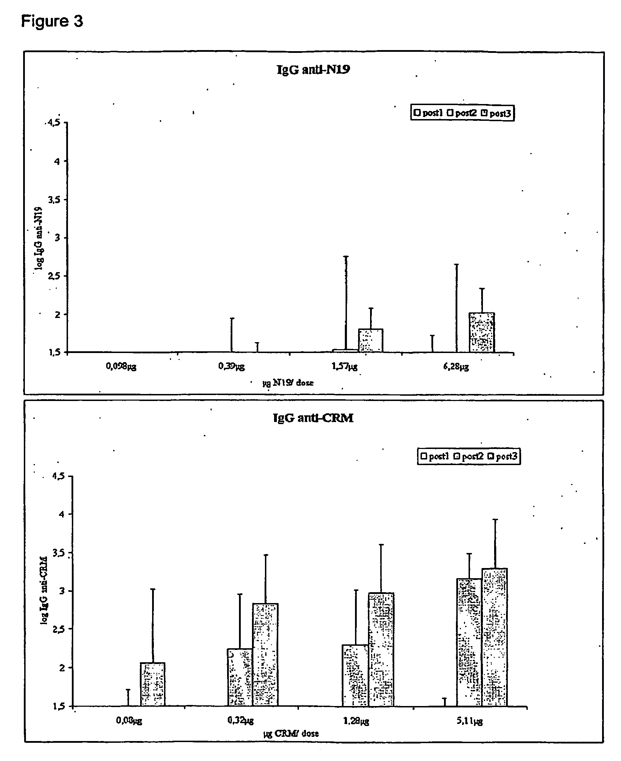

Figure 2 shows serum anti-MenC IgG antibody responses. Groups of six BALB/c mice were immunized three times with decreasing amounts of N19-MenC or CRM-MenC (2.5, 0.625, 0.156 and 0.039 µg of MenC/dose) and 0.5 mg of aluminuim hydroxide. Serum samples were collected before (pre) and after (post-1, -2, and -3) each immunization and tested individually to quantitate MenC-specific IgG antibody titers. Each point represents the mean antibody titer (±1 SD) of each group at each time point. Figure 3 shows anti-carrier IgG antibody responses in single serum samples of mice immunized as described before. Since mice were immunized with equal amounts of MenC in either conjugate, the final amount of carrier protein is slightly different in the groups receiving the CRM-MenC and those that received the N19-MenC, due to the slight difference in the sugar-to-protein ratios in the two constructs. Serum samples were collected before (pre) and after (post-1, -2, and -3) each immunization and tested individually to quantify carrier-specific IgG antibodies. Each point represents the mean antibody titer (± SD) of each group at each time point.

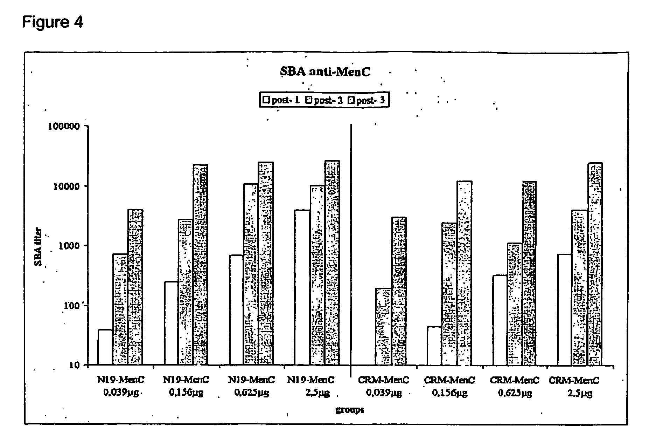

Figure 4 shows bactericidal activity in serum samples of mice immunized three times with decreasing amounts of N19-MenC or CRM-MenC (2.5 µg, 0.625 µg, 0.156 µg, 0.039 µg of MenC/ dose) and 0.5 mg of aluminum hydroxide. Bactericidal antibody titers from pooled serum samples collected before (pre) and after (post- 1, -2, and -3) each immunization are shown. Results were expressed as reciprocal values of the highest serum dilution giving more than 50% bacterial killing.

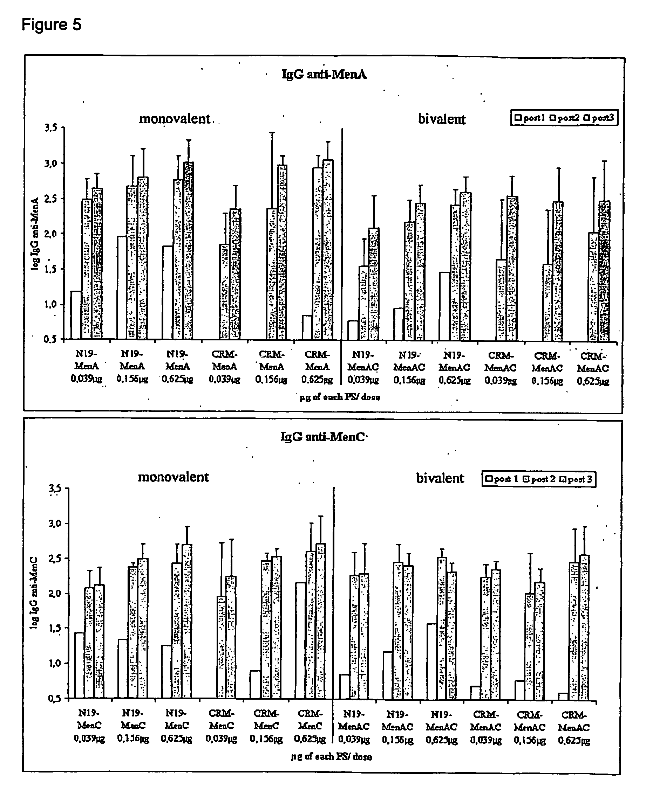

Figure 5 shows serum anti-MenA and anti-MenC antibody responses. Groups of six BALB/c mice were immunized three times with decreasing amounts of N19-MenA and N19-MenC either alone or combined, or CRM-based conjugates (0.625, 0.156 and 0.039µg of MenA and/or MenC/ dose) in the presence of 0.06 mg of aluminum phosphate. Serum samples were collected before (pre) and after (post-1, -2, and -3) each immunization and anti-MenA and MenC-specific IgG antibody titers were measured. Each point represents the mean antibody titer (±1 SD) of each group at each time point.

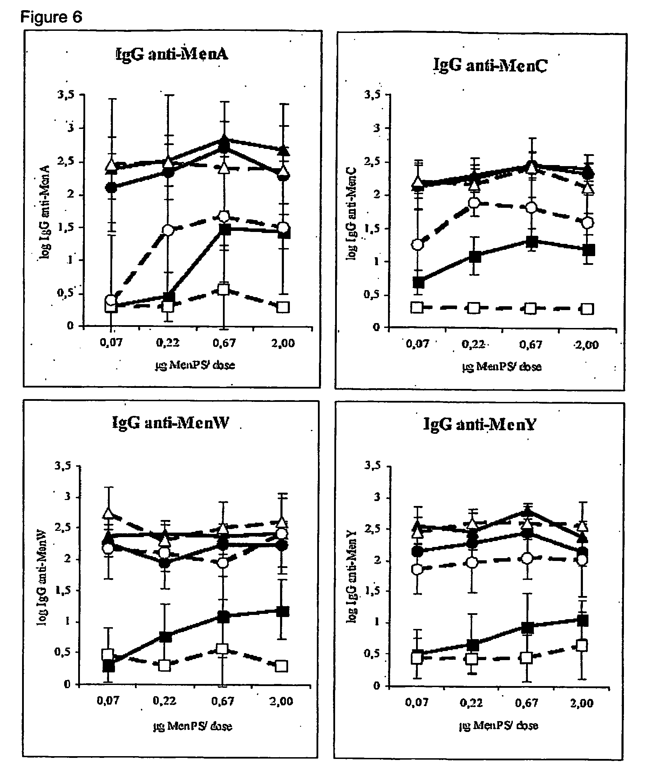

Figure 6 shows the effect on serogroup specific antibody responses of a dose escalation of N19 tetravalent combined conjugate vaccines. Groups of six BALB/c mice were immunized with decreasing amounts of N19-MenACWY (continuous lines) or CRM-MenACWY (broken lines) (from 2 to 0.074 µg of each MenPS/ dose) in the presence of 0.06mg of aluminum phosphate as adjuvant. Immunizations were performed on day 0, 21 and 35 and serum anti-MenA, anti-MenC, anti-MenW and anti-MenY specific IgG antibody titers were measured after each immunization (post -1, -2 and -3). Each point represents the mean antibody titer (± 1SD) of each group at each time point.

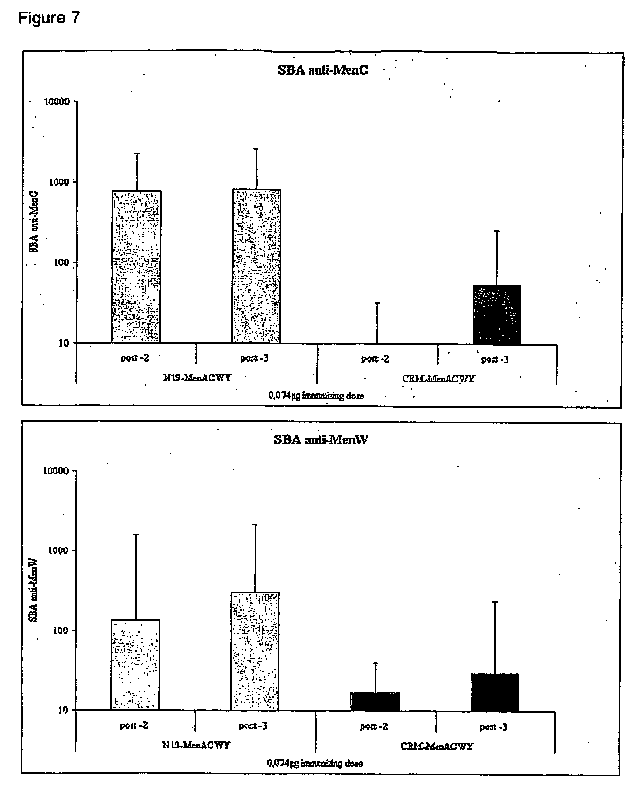

Figure 7 shows bactericidal activity against group C and W-135 in single sera obtained from mice after two (post -2) and three (post -3) immunizations with 0.074µg of each PS per dose (N19-MenACWY or CRM-MenACWY). Titers are expressed as reciprocal values of the highest serum dilution giving at least 50% bacterial killing. Each column represents mean titers (± SD) of the group at each time point.

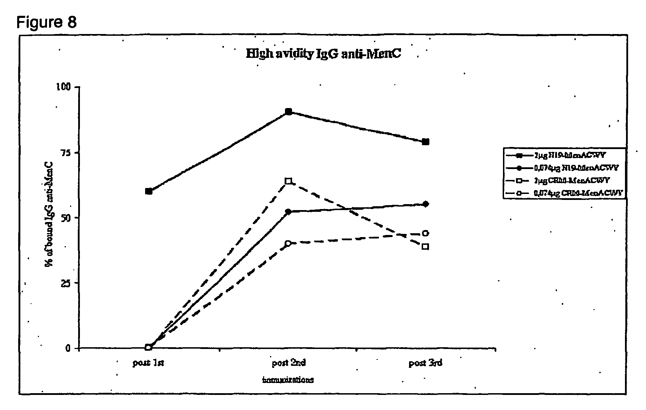

Figure 8 shows dynamics of the avidity profile of anti-MenC antibodies generated in mice after immunization with N19-MenACWY or CRM-MenACWY as detailed in Materials and Methods.
High avidity IgG titers were measured on pooled sera by a modified ELISA method. Results are expressed in avidity index (AI) corresponding to the percentage of bound antibodies after elution with 75mM of NH₄SCN of each group after each immunization (post 1st, post 2nd, post 3rd).

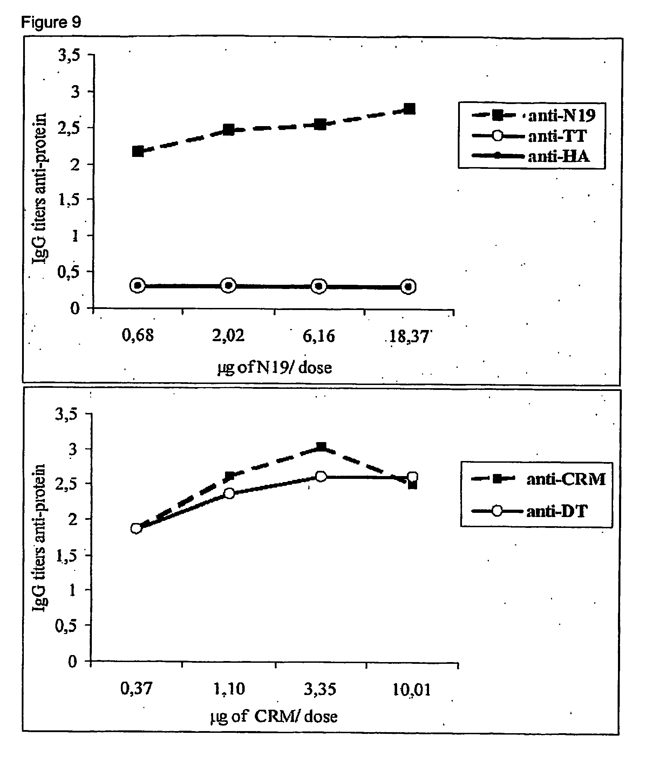

Figure 9 shows antibody responses against the carriers and its parent proteins in pooled sera obtained after the third immunization. Each point represents the antibody titer of each group after three immunizations as described before.

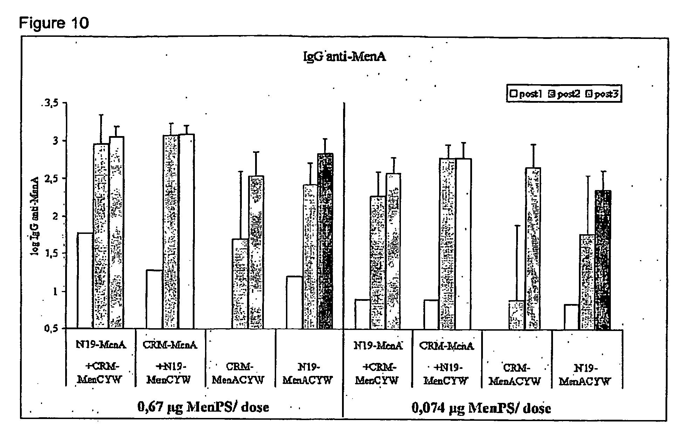

Figure 10 shows serum anti-MenA antibody responses. Groups of six BALB/c mice were immunized three times with decreasing amounts of tetravalent formulations prepared mixing together MenA conjugated either to N19 or CRM with MenCWY conjugated either to CRM or N19 (N19-MenA+CRM-MenCWY and vice versa CRM-MenA+ N19-MenCWY). Control groups received tetravalent formulations containing one carrier (N19-MenACWY or CRM-MenACWY). Mice received decreasing amount of tetravalent formulations (from 0.67 µg to 0.074 µg of each MenPS/ dose) in the presence of 0.06 mg aluminum phosphate as adjuvant. For simplicity we report only the results obtained after the highest (0.67 µg) and the lowest (0.074µg) immunizing dosage.

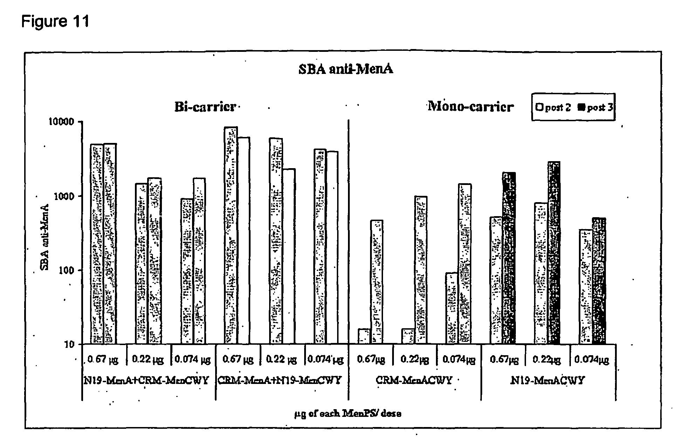

Figure 11 shows serum bactericidal activity of BALB/c mice immunized three times with decreasing amount of bi-carrier or mono-carrier formulations as described above. Bactericidal antibody titers from pooled serum samples collected after the second (post -2) and the third (post- 3) immunization were measured. Results were expressed as reciprocal values of the highest serum dilution giving more than 50% bacterial killing.

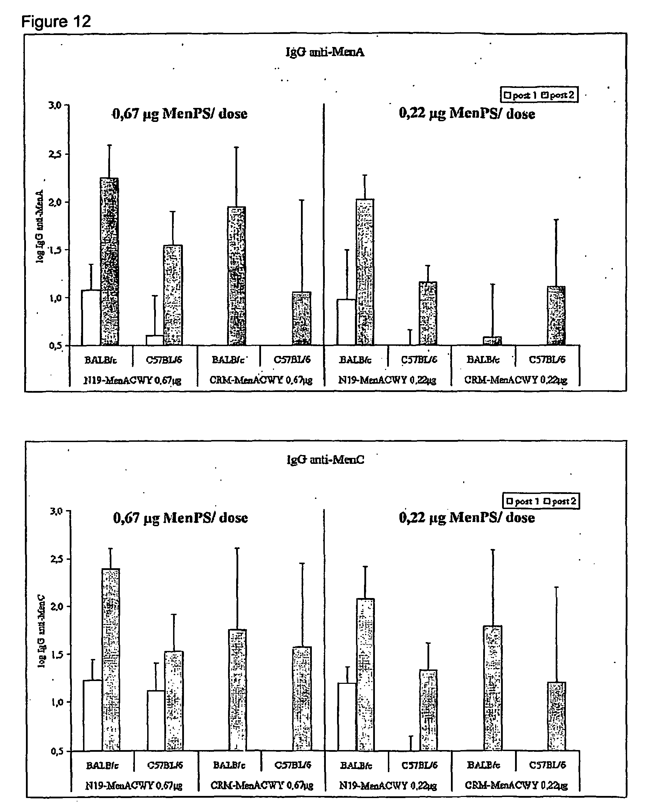

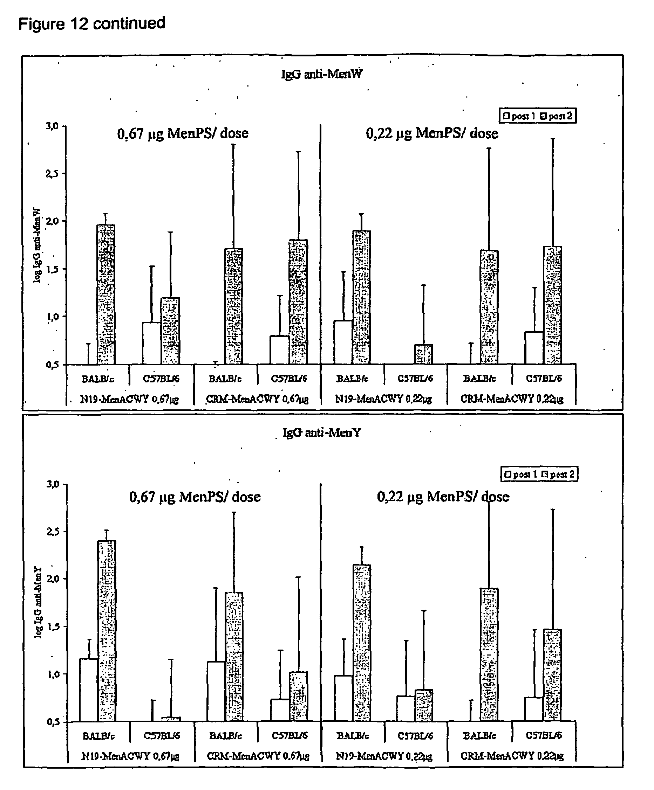

Figure 12 shows anti-capsular IgG antibody responses. Groups of BALB/c or C57BL/6 mice were immunized twice with N19-MenACWY or CRM-MenACWY (0.67 or 0.22 µg/ dose of each MenPS) conjugates in the presence of 0.06 mg of aluminum phosphate. Serum samples were collected before (pre) and after (post-1 and -2) each immunization and MenA, MenC, MenW, MenY-specific IgG antibody titers were measured. Each point represents the mean antibody titer (±1 SD) of each group at each time point.

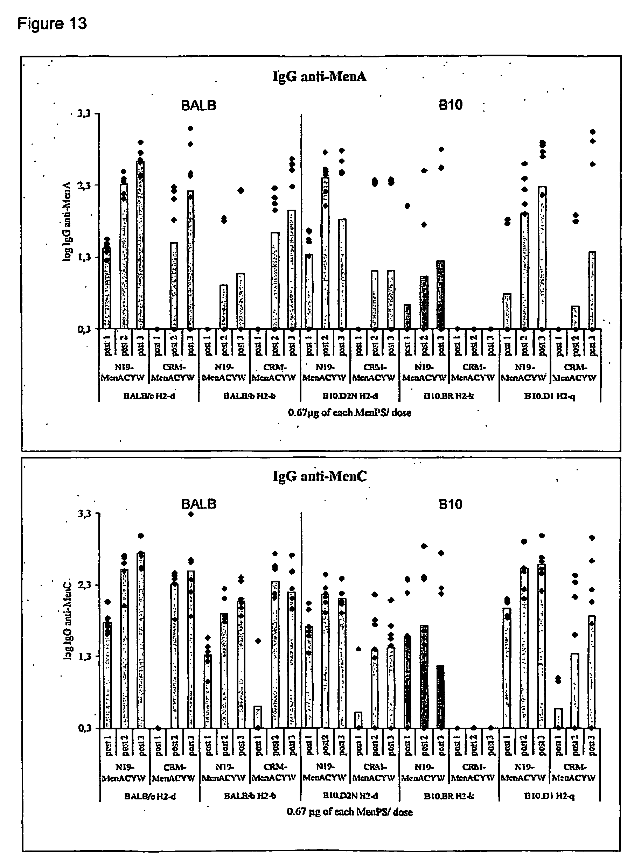

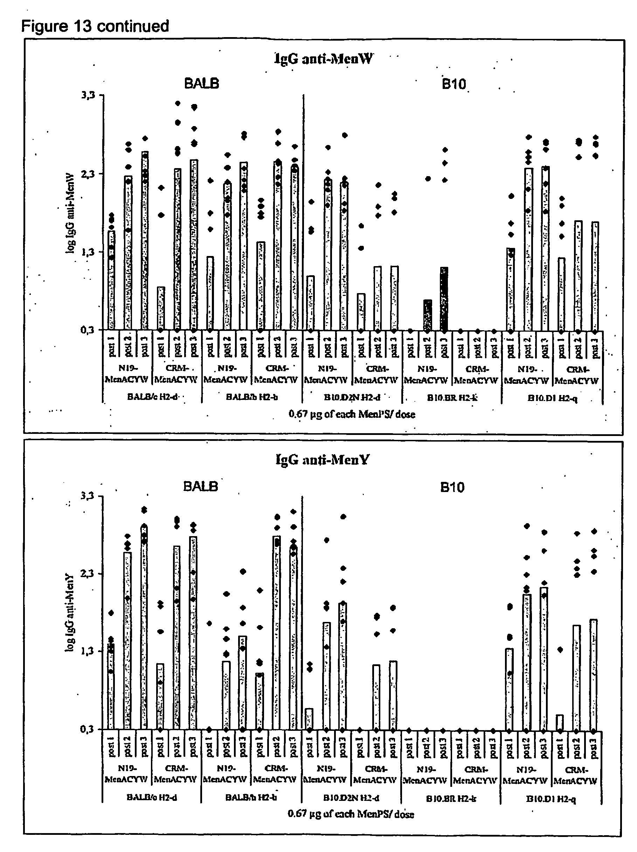

Figure 13 shows anti-capsular IgG antibody responses. BALB/c H-2 d , BALB/B H-2 b, B10.BR H-2 k , B10.D2N H-2 q and B10.D1 H-2 d mice were immunized three times with N 19-MenACWY or CRM-MenACWY (0.67 µg/ dose of each PS) in the presence of 0.06 mg of aluminum phosphate. Serum samples were collected before and after (post-1, -2 and -3) each immunization and MenA, MenC, MenW, MenY-specific IgG antibody titers were measured.
Each bar represents the mean antibody titer and symbols correspond to the single mouse of each group at each time point.

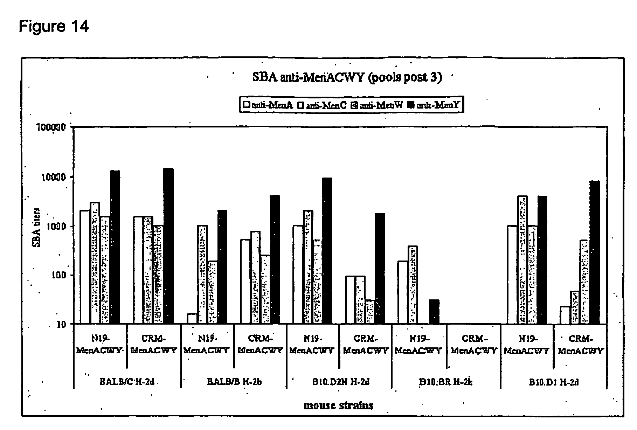

Figure 14 shows serum bactericidal activity of mice with different genetic background immunized three times with N19-MenACWY or CRM-MenACWY (0.67 µg of each MenPS/ dose) and 0.06 mg of aluminum phosphate. Bactericidal antibody titers from pooled serum samples collected after the third immunization (post-3) are shown. Results were expressed as reciprocal values of the highest serum dilution giving more than 50% bacterial killing.

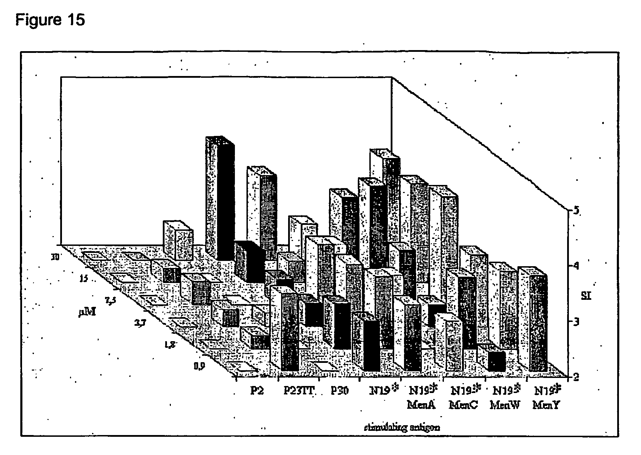

Figure 15 shows N19 epitope-specific T cell proliferation responses. Spleen cells from mice immunized three times with N19-MenACWY (6 µg N19/ dose) were tested to proliferate *in vitro* in the presence of 0.9- 30 µM of three individual peptides (P2TT, P23TT, P30TT) and 0.312 to 10 µg/ ml of N19 protein, free or conjugated to the PSs as indicated in the graph. Results were expressed as stimulation index (SI) = (cpm experimental/ cpm background unstimulated). *=N19 concentrations from 0.312 to 10 µg/ml

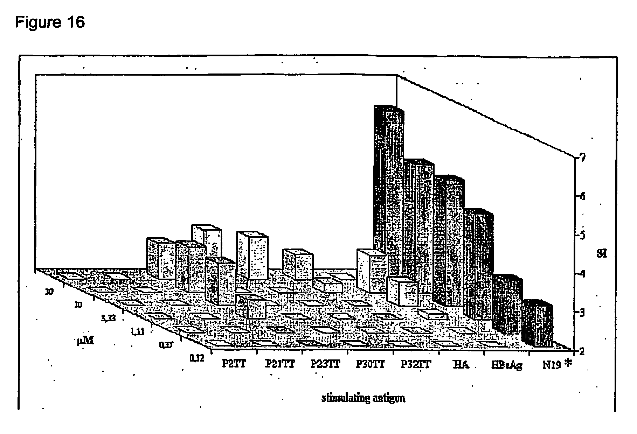

Figure 16 shows N19 epitope-specific T cell proliferation responses. Spleen cells from mice immunized twice with N19-MenACWY (6 µg N19/ dose) were tested to proliferate *in vitro* in the presence of 0.12-30 µM of individual peptides (P2TT, P21TT, P23TT, P30TT, P32TT, HA, HBsAg) and 0.004 to 1 µM of N19 as indicated in the graph. Results were expressed as stimulation index (SI) = (cpm experimental/ cpm background unstimulated). *= N19 concentrations from 1 to 0.004µM

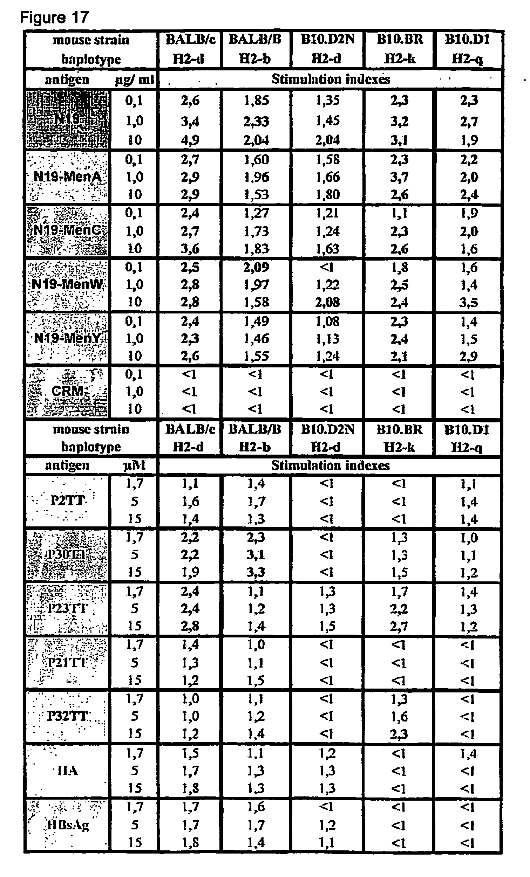

Figure 17 shows T- cell proliferative response of congenic strains of mice immunized with N19-MenACWY. Strains of mice with different H-2 haplotype were immunized three times with N19-MenACWY (6 µg N19/dose) in the presence of 0.06 mg of aluminum phosphate. Spleen cells were tested to proliferate *in vitro* in the presence of 1.7- 15 µM of N19 peptides (listed in table 1) and 0.1 to 10 µg/ ml of N19 protein, free or conjugated to the MenPSs. Results were expressed as stimulation index (SI) = (cpm experimental/ cpm background unstimulated). A SI >2 was considered positive.

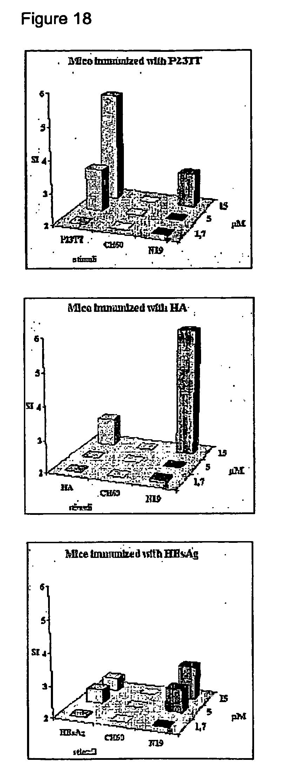

Figure 18 shows T cell activation specific for P23TT, HA and HBsAg. Stimulation indexes to homologous peptides and N19 protein, as determined in proliferation assay. Groups of three mice were immunized at the base of the tail with 50 µl volume containing 50 µg of individual peptide emulsified 1:1 in CFA. Seven days later lymph nodes were removed and LN cells tested for their capacity to proliferate in the presence of the homologous peptide or N19 protein at different concentrations. Results were obtained in triplicate cultures of single mouse. Results were expressed as stimulation index (SI) calculated from average cpm of the experimental group/ cpm background.

### MODES FOR CARRYING OUT THE INVENTION

### 1. Glycoconjugate preparation

### 1.1 Expression and purification of the polyepitope protein N19.

*E. coli* strains carrying the recombinant plasmids pQE-N19 were grown O/N on LB-agar plates, 100 µg/ml ampicillin at 37°C. The grown bacteria were then inoculated in 500 ml LB medium, 100 µg/ml ampicillin and grown O/N at 37°C. The 500 ml were then diluted in 5 1 medium in a fermentator. The growth has been conducted in optimised conditions. When an OD₆₀₀ₙₘ value of 4.2 was obtained, the expression of the polyepitope protein was induced for 3.5 hours by adding 1 mM IPTG (iso-propyl-thio-galactoside) until an OD₆₀₀ₙₘ 7.2. Two samples of the bacterial culture supernatant were collected, at time zero before adding IPTG (to OD 4.2) and the end time point of expression (t_{end} OD 7.2). The pellet obtained was resuspended in sample buffer and loaded onto a 12.5% SDS-PAGE in serial dilution corresponding to different bacterial culture ODs. The whole bacterial culture was centrifuged at 5000 g in a JA10 rotor (Beckman, Fullerton, CA) for 20 min at 4°C. The cellular pellet obtained of 60g was suspended in 500ml lysis buffer (6 M guanidine-HCl, 100 mM NaH₂PO₄, 2 mM TCEP (Pierce) pH 8, stirred for 1 h at RT and then incubated for 1 h at 37°C. The supernatant containing the dissolved protein was collected by centrifugation at 12000 rpm in a J20 rotor (Beckman) for 20 min at RT and subjected to Immobilized Metal Affinity Chromatography (IMAC). Before adsorbing the sample on the IMAC column, 1 mM TCEP (Tris (2-carboxyethyl) phosphine hydrochloride, Pierce) had been added, which showed previously to be essential during the purification, to avoid co-purification of contaminating substances bound covalently to N19 by disulphide bonds. The dissolved material was loaded onto a XK50 column containing 360 ml of Nickel activated IDA (iminodiacetic acid) Chelating Sepharose Fast Flow (Pharmacia, Uppsala, Sweden), the column was then washed with 5 volumes of lysis buffer. Then a 300 ml gradient was applied from guanidine-HCl 6 M pH 8 to urea 8 M pH 8 containing 1 mM TCEP. The column was washed with 3 volumes of buffer B (8 M urea, 100 mM NaH₂PO₄, pH 7) and the proteins were eluted with 1800 ml 0-200 mM imidazole gradient in buffer B. Fractions collected from the column were qualitatively analyzed on 12.5% SDS-PAGE (BioRad) and quantitatively by Bradford protein determination method (BioRad protein assay).

The selected gradient fractions containing the purified recombinant proteins were subjected to Cation Exchange Chromatography (CEC). The 600 ml pooled fractions were loaded on a XK50 column containing 120 ml SP-Sepharose Fast Flow resin (Pharmacia, Uppsala, Sweden). The column was washed with 5 volumes of buffer C (7 M urea, 20 mM NaH₂PO₄ pH 7, 10 mM β-Mercaptoethanol) and the proteins were eluted with 1300 ml 0-500 mM NaCl gradient in buffer C. The gradient fractions containing the purified recombinant proteins, selected by 12.5% SDS-PAGE analysis, (BioRad) were pooled and dialyzed against 10 mM NaH₂PO₄, 150 mM NaCl, 10% glycerol. The final protein concentration was determined by the micro BCA method according to the manufacturer's instructions (Pierce). Protein was analyzed on 12.5% SDS-PAGE (BioRad). Optical density of the bands has been measured for integrity evaluation (Image Master 1D Elite v4.00 LabScan Computer Program). The level of endotoxins in the final protein preparation was determined by the kinetic turbidimetric method of the limulus amebocyte lysate (LAL) by Quality Control Department (Chiron Vaccines Siena).

### 1.2 Production of oligosaccharides.

The group A, C, W, Y meningococcal polysaccharides were purified from *Neisseria meningitidis* strains by the standard procedure described for meningococcal vaccine production (291). Purified capsular polysaccharides were then depolymerised and activated in order to be coupled to the carrier protein as previously described (292, 293). Briefly we describe here the procedure for meningococcal serogroup C oligosaccaride preparation. The purified MenC capsular polysaccharide was submitted to hydrolysis in 10 mM sodium acetate buffer pH 5.0 to reduce the average degree of polymerization (DP). The reaction is conducted at 80°C for ~12 h until a DP of 10 was reached. The DP can be followed on-line during the hydrolysis by analysing total sialic acid content in the starting polysaccharide solution (constant during hydrolysis) and formaldehyde released from the terminal group of each chain after oxidation. This real-time DP measurement permitted the extrapolation of the end time of the hydrolysis. Oligosaccharides were sized by Q-Sepharose FF ion-exchange chromatography that retained the higher molecular weight polysaccharides on the column while the low molecular weight oligosaccharides (DP <6) were eluted from the column with 5 mM sodium acetate buffer, 100 mM in NaCl, pH 6.5. The desired oligosaccharide fraction was then eluted with 0.7 M tetrabutylammonium bromide (TAB), a positive counterion, which displaced the negatively charged oligosaccharides from the column. The products were then submitted to concentration/ diafiltration against water on a 3K cut-off membrane to remove the excess of TAB and to concentrate the MenC oligosaccharide in preparation. After the diafiltration to retentate was dried by a rotary evaporation step. Thereafter the MenC oligosaccharide was subjected to reductive amination to yield an oligosaccharide with a terminal primary amino group. The reaction mixture was made up to 10% DMSO, 90% methanol, 50 mM ammonium acetate and 10 mM sodium cyanoborohydride and incubated for 24 h in a covered water bath at 50°C. The reaction mixture was then submitted to a rotary evaporation step to reduce the methanol content of the amination reaction mixture to avoid possible interaction with silicon tubing and diafiltration membranes in the following diafiltration step. The aminated oligosaccharides were then purified from reagents (cyanoborohydride, DMSO, methanol) by concentration/ diafiltration against 8 volumes of 0.5 M NaCl, followed by 4 volumes of 20 mM NaCl. The purified aminated oligosaccharides were dried under vacuum in preparation for the activation step. The MenC oligosaccaride was solubilized in water followed by the addition to the mixture of DMSO. Triethylamine (TEA) was added to ensure sufficient deprotonation of the oligosaccharide primary amino group and of the di-N-hydroxysuccinimide (bis-NHS) ester of adipic acid. The bis-NHS was addded in molar excess to favor the formation of the covalent linkage of a single oligosaccharide polymer to each molecule of bis-NHS ester. The activated oligosaccharide was precipitated by addition of acetone to the reaction mixture, which was also used to separate the oligosaccharides from DMSO, bis-NHS ester and the TEA. The precipitate was dried under vacuum, weight and stored at 20°C until the use for conjugation.

The procedure for purification of the other PSs was basically the same with minor modifications in the reaction time and temperature [294].

### 1.3 N19 conjugation to meningococcal oligosaccharides.

After purification, sizing and activation oligosaccharides were used for the subsequent conjugation to N19 protein [295]. Before starting the conjugation experiment we evaluated preliminarily the potential aspecific adsorption of the polysaccharides to the Ni-activated resin. In a typical conjugation experiment, 343.2 nmol of N19 carrier protein was dissolved in Guanidium-HCl pH 8, 100 mM Na₂HPO₄, and adsorbed to a previsouly packed 5 ml Ni-activated Sepharose Fast Flow resin (Pharmacia, Uppsala, Sweden) equilibrated in the same buffer. Guanidinium-HCl was removed by washing the resin with 50 ml of 100 mM phosphate buffer pH 7.5 and then 1 ml of 100 mM phosphate buffer pH 7.5 containing 6864 nmol of activated meningococcal oligosaccharide (MenA, MenC, MenW or MenY) was added to the column, recirculating at room temperature for 2 h. The column was washed with 50 ml of 100mM Na₂HPO₄ pH 7.5 to remove the excess of unconjugated oligosaccharide. Finally, the conjugate product was eluted with 300 mM imidazole, pH 7, 100 mM NaH₂PO₄ and analyzed on 7.5 % SDS-PAGE. The selected fractions containing the conjugate were pooled and dialyzed against PBS. The glyco-conjugates were analyzed for sugar and protein content. The saccharide content of MenC, MenW and MenY conjugates was quantified by sialic acid determination (143), while that of MenA conjugate by mannosamine-1-phosphate chromatographic determination (121). The protein content was measured by micro BCA assay (Pierce, Rockford, IL). The glycosylation degree was calculated from the sugar-to-protein ratio in weight. The CRM-based conjugate vaccines (CRM-MenA, CRM-MenC, CRM-MenW, CRM-MenY) taken as reference in this study were prepared by the Manufacturing Department (Chiron Vaccines Siena). *2. Mouse strains.*

Unless otherwise specified groups of six female 7-week old mice BALB/c were used. In another experiment, four congenic strains of 7-week old female mice with the following H-2 haplotype were used: BALB/B (H-2^{b}) congenic with BALB/c (H-2^{d}) and B10.BR (H-2^{k}), B10.D2N (H-2^{q}), B10.D1 (H-2^{d}) congenic with C57BL/6 (H-2^{b}). The mice were purchased from Charles River (Calco, Italy) or from Jackson Laboratories (Bar Harbor, Maine).

### 3. Mouse immunization schedules and formulations.

Mice were immunized subcutaneously on days 0, 21 and 35 with N19 or CRM conjugates with different 0.5 ml formulations of monovalent, bivalent, tetravalent or bi-carrier conjugate vaccine based on saccharide content diluted in NaCl 0.9% buffer as specified below. Individual serum samples were taken at days -1 (pre), 20 (post- 1), 34 (post- 2) and 45 (post- 3) and frozen at -20°C until use. Spleens were collected from mice immunized with N19-conjugates for assessing T-cell proliferation as described in cell-mediated immune response section.

### 3.1 Monovalent meningococcal C conjugate vaccine.

Mice were immunized with decreasing amounts of N19-MenC or CRM-MenC (from 2.5 to 0.039 µg of MenC/ dose) in the presence of 0.5 mg aluminium hydroxide as adjuvant. Antibody titres were measured as detailed below.

The conjugate containing N19 was more immunogenic than the one with CRM (Figure 2). After two immunizations the N19-based constructs induced serum anti-MenC IgG antibodies at titers significantly higher than those induced by three doses of the CRM-MenC conjugate (*e.g*. post- 2 N19-MenC at 0.625 µg versus post- 3 CRM-MenC at 0.625µg [P < 0.01]; post-2 N19-MenC at 0.156µg versus post- 3 CRM-MenC at 0.156µg [P < 0.05]). In addition after three doses, lower amounts of N19 conjugate were enough to induce anti-MenC IgG antibodies significantly higher than those induced by the CRM-MenC conjugate (*e.g*. N19-MenC at 0.156µg versus CRM-MenC at 0.625µg [P < 0.01]).

Two and three immunizations with CRM-based conjugates induced strong anti-carrier antibody responses against CRM even at the lowest doses tested (*i.e*. 0.3 µg and lower). On the contrary, the N19-specific antibody response was always negligible and was detectable (even though at very low titers) only at the highest dose (i.e*.* 6 µg) (Figure 3). These low-titer anti-N19 antibodies did not recognize tetanus toxoid in solid phase. These results clearly show that the strong helper effect of the N19 polyepitope is not accompanied by the induction of significant levels of antibodies to itself nor to native proteins.

Since protective immunity against MenC relies mainly on bactericidal antibodies that kill the bacteria in the presence of complement, the functional activity of the antibodies induced was measured. In agreement with the results obtained in ELISA, Figure 4 shows that N19 conjugates were able to induce bactericidal antibodies at immunizing doses lower than those used with CRM-based conjugates. It is noteworthy that following one immunization at the highest dose the N19-MenC conjugate induced bactericidal antibodies with titers similar to those induced by two doses of the CRM-MenC conjugate. Mice immunized twice with lower amounts of N19-MenC produced higher bactericidal antibody titers, than those immunized with CRM-MenC. These CRM-MenC immunized mice required a third dose to reach bactericidal antibody titers, comparable to those induced by N19 conjugates. Therefore, N19 showed to behave as a stronger carrier than CRM by inducing antibodies with substantial functional activity against MenC after less injections with less dosage.

### 3.2 Bivalent meningococcal AC conjugate vaccine.

Mice were immunized N19-MenA and N19-MenC separately and combined or CRM-MenA and CRM-MenC separately and combined (0.625, 0.156 or 0.039 µg of each MenPS/ dose) in the presence of 0.06 mg aluminum phosphate as adjuvant. Antibody titres were measured by ELISA as described below.

As shown in upper panel in Figure 5, administering together MenA and MenC conjugates containing either N19 or CRM carrier the immunogenicity against MenA was accompanied as expected by a significant reduction compared to that of single given conjugates (e.g. at 0.156 µg post- 2 N19-MenA versus N19-MenAC [P < 0.05]; at 0.625 µg post-3 N19-MenA versus N19-MenAC [P < 0.05]; at 0.625µg versus post- 2 CRM-MenA versus CRM-MenAC [P < 0.05]; at 0.156µg versus post- 3 CRM-MenA versus CRM-MenAC [P < 0.05]). Nevertheless, both bivalent formulations containing either N 19 or CRM carrier induced comparable (no statistically different) antibody titers against MenA after two and three immunizations. N19 carrier in mono-and bivalent conjugate vaccines was able to induce a faster antibody response against MenA, raising an antibody response already after the first dose, while CRM conjugates didn't induce any measurable titer of antibodies. Also after two injections of N19 conjugates the trend was to elicit higher antibody response than CRM conjugates, but the differences became statistically significant only at the lowest given dosage of the monovalent vaccine (*e.g*. at 0.039 µg post- 2 N19-MenA versus CRM-MenA [P < 0.05]).

When the anti-MenC antibody response was measured (lower panel in Figure 5), no decrease of the titers after two or three doses was observed when the monovalent and bivalent formulations were compared. After one administration lowering the immunizing dosage of monovalent vaccine anti-MenC antibody levels obtained with CRM conjugates were abrogated, while those obtained with N19 conjugates maintained stable. Comparing titers obtained after one immunization with the bivalent vaccines, CRM conjugates showed to be unable to raise a substantial anti-MenC antibody response, while N19 conjugates induced higher levels with a dose-response behavior.

### 3.3 Tetravalent meningococcal ACWY conjugate vaccine.

Tetravalent formulations were prepared mixing together in equivalent saccharide amount N19-MenA, N19-MenC, N19-MenW and N19-MenY (N19-MenACWY). As reference we used clinical grade lots of CRM conjugate vaccine (Chiron Vaccines, Siena) formulated before use by mixing liquid CRM-MenCWY to lyophilised CRM-MenA. Mice received decreasing amounts of tetravalent formulations (from 2 µg to 0.074 µg of each MenPS/ dose) in the presence of 0.06 mg aluminum phosphate as adjuvant.

Figure 6 shows for any of the four serogroup capsular polysaccharides and at all given dosages that two or three immunizations with N19-MenACWY produced similar IgG titers. When comparing the antibody responses to the CRM conjugates after three immunizations and those to N19 conjugates after only two immunizations, no significant differences were found for all four serogroups. After the second dose, the antibody titers against serogroups A and C when conjugated to N19 were significantly higher compared to those obtained when conjugated to CRM (IgG anti-MenA and anti-MenC: post -2 at all given dosages N19 versus CRM: P < 0.05).

N19 conjugates induced antibody production against all four polysaccharides after primary immunization, while CRM conjugates did not. In particular against MenC, as shown in panel B of Figure 6, significantly higher antibody titers were obtained with N19 conjugates at all given dosages (post -1 at all given dosages N19 versus CRM: [P < 0.05]). Titers against MenA and MenW, shown in panel A and C, were significantly higher at the highest dosage when N19 conjugates were given once (at 2µg post -1 N19 versus CRM: [P < 0.05]). Antibodies induced by both conjugates were predominantly IgG1 (data not shown). Importantly, we noticed that the number of responder mice was higher when immunized with N19 conjugates than with CRM conjugates, especially after the first and the second dose, while after the third dose all mice responded (Table 2).

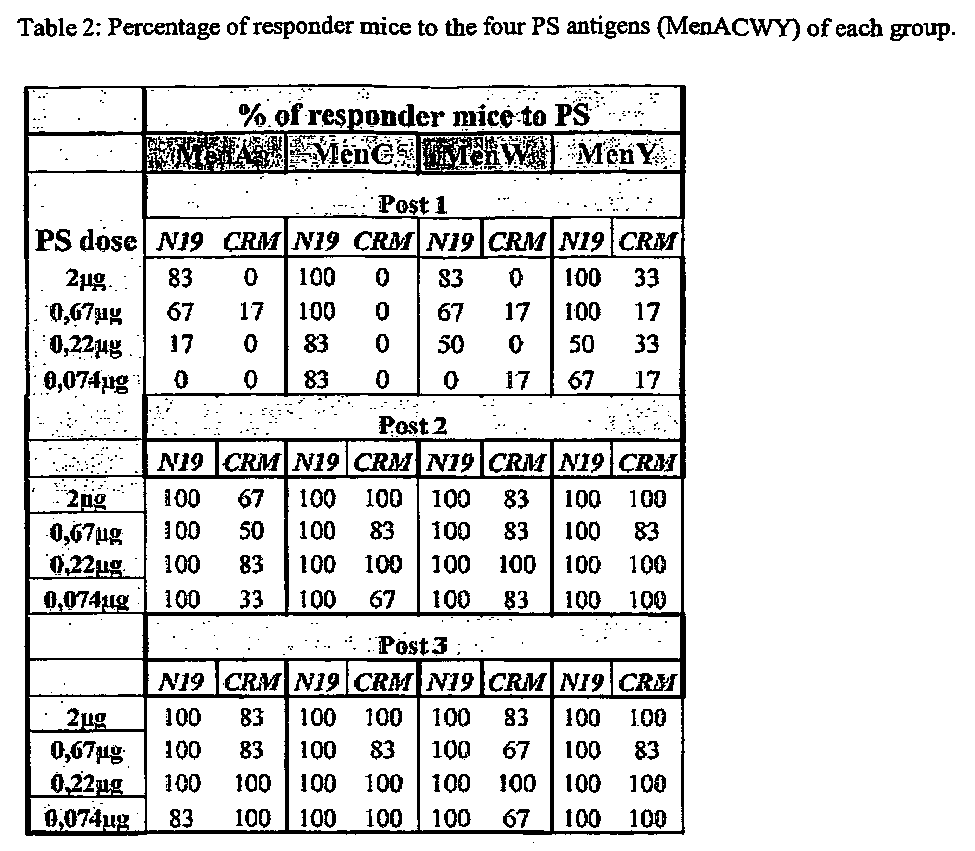

N19-MenACWY was highly effective in inducing bactericidal antibodies against all four Men polysaccharides. In particular, bactericidal titers against group C were significantly higher at all given dosages after two doses of N19 conjugates than of CRM conjugates. Performing a dose escalation, the potency of N19 carrier was highlighted, since limiting the dose, N19 conjugates induced higher bactericidal antibody titers against all four polysaccharides than those induced by CRM conjugates. Bactericidal titers against MenC and MenW on single sera from mice immunized with the lowest dose (0.074µg) were analysed in particular. Figure 7 shows that as for ELISA titers, also the Serum Bactericidal Antibody (SBA) titers obtained with N19 conjugates were comparable after two or three doses. Bactericidal titers against MenC were already significantly higher after two immunizations with N19 conjugates than those obtained after three injections of CRM conjugates (SBA anti- MenC: post -2 N 19 versus post -3 CRM: [P < 0.05]). Comparing bactericidal titers against MenW after two doses or after three obtained either with N19-based or with CRM-based conjugates, we found that N19 conjugates induced significantly higher bactericidal antibody titers (SBA anti- MenW post -2 N19 versus CRM: [P < 0.05]; post -3 N19 versus CRM: [P < 0.05]).

A detailed analysis of functional activity of group A and C antibodies was conducted using a modified antigen-binding assay that measures only high affinity antibodies [296]. Results show in Figure 8 that the antibodies obtained against MenC with 2 µg of N19 conjugates were already of high avidity after one dose. Two immunizations were sufficient to induce an efficient avidity maturation of almost all the antibodies. The other groups, immunized either with lower amounts of N19 conjugates or with CRM conjugates, showed a similar maturation profile, with an increase from the baseline to about 50% of high avidity antibodies only after two doses (for simplification only groups immunized with the highest and the lowest dosages are shown).

To evaluate the influence of the carrier protein shared by four polysaccharides to induce antibodies against itself, we measured antibodies against both carrier proteins employed (Figure 9). In addition, we analyzed whether the produced antibodies against the carriers were able to bind also parent proteins. Figure 9 shows in panel A that antibodies produced with CRM conjugates equally well recognized DT, CRM's parent protein. On the contrary, antibodies to N19 conjugates did not cross-react with its parent proteins, such as tetanus toxoid (TT) and influenza haemagglutinin (HA), from which N19 epitopes were derived. It should be noted that ten epitopes (five repeated twice) from TT are contained in N19, representing more than 50% of its sequence.

### 3.4 Bi-carrier tetravalent meningococcal ACWY conjugate vaccine.

Tetravalent formulations were prepared mixing together MenA conjugated either to N19 or CRM with MenCWY conjugated either to CRM or N19 (N19-MenA+CRM-MenCWY and vice versa CRM-MenA+ N19-MenCWY). Control groups received tetravalent formulations containing one carrier (N19-MenACWY or CRM-MenACWY). Mice received decreasing amounts of tetravalent formulations (from 0.67 µg to 0.074 µg of each Men polysaccharides/ dose) in the presence of 0.06 mg aluminium phosphate as adjuvant. Antibody titres were determined using the methods described below.

N19-MenACWY produced, after the first dose, anti-MenA titers comparable to those obtained after two doses of CRM based vaccine (Figure 6). Moreover, mice immunized twice with N19 conjugates elicited significantly higher bactericidal titers against MenA, than those immunized with CRM conjugates (Figure 10). We observed that when N19-MenA was administered simultaneously with CRM-MenCWY or vice versa interchanging the carrier on MenA, the antibody response was significantly increased compared to tetravalent formulation containing a unique carrier (*e.g*. post-2 at 0.67 µg: N19-MenA+CRM-MenCWY versus N19-MenACWY: [P <0.05]; post -3 at 0.67 µg: N19-MenA+ CRM-MenCWY versus CRM-MenACWY: [P <0.01]; post -2 at 0.22 µg: CRM-MenA+N19-MenCWY versus CRM-MenACWY: [P <0.001]). However, we noticed that lowering the immunizing dosage of both bi-carrier formulations, the anti-MenA antibodies decreased significantly in the IgG titer but not in their bactericidal titer neither after two nor after three immunizations (Figure 10). Moreover, both bi-carrier vaccines induced comparable bactericidal titers at all dosages (Figure 11). It is noteworthy that the presence of N19 in all the formulations evoked consistently an antibody response after only one immunization, while CRM alone did not (Figure 10).

### 3.5 Mouse strains with different genetic background.

In a preliminary experiment, two groups of mice BALB/c and C57BL/6 were immunized twice with 0.67 or 0.22 µg N19-MenACWY or CRM-MenACWY with of 0.06 mg aluminium phosphate. In another experiment, congenic strains of mice were immunized three times with tetravalent formulations N19-MenACWY or CRM-MenACWY (0.67 µg of each Men polysaccharide/ dose) in the presence of 0.06 mg phosphate prepared as described above. BALB/c mice were used as control.

Based on the above results obtained in BALB/c mice, we decided to immunize mice only twice with two different dosages of tetravalent formulations containing N19 or CRM and the antibody responses against the four polysaccharides were measured (Figure 12). Again, it was evidenced in BALB/c mice that N19 behaved as stronger carrier than CRM in the tetravalent vaccine in particular in inducing anti-MenA antibodies (BALB/c 0.22µg post 2 N19 versus CRM: [P<0.001]). We observed that both conjugates containing either N19 or CRM were less immunogenic in C57BL/6 than in BALB/c mice, and the antibody responses were more variable. Furthermore the better carrier effect of N19 was less evident against all four polysaccharides than that observed in BALB/c. Nevertheless, N19 conjugates were capable to elicit consistently antibody titers against all four polysaccharides already after the first immunization, while CRM conjugates were not.

As shown in Figure 13, N19 and CRM conjugates were more immunogenic against the four conjugates in BALB/c H-2^{d} and B10.D1 H-2^{q} strains. In general, more mice responded when immunized with N19-based than with CRM-based conjugates. B10.D2N H-2^{d} with the same haplotype as BALB/c mice, were better recipients for N19- than for CRM-conjugates. On the one hand, BALBB H-2^{b} congenic with BALB/c mice were better recipients for CRM-, than for N19-conjugates. On the other hand, CRM conjugates did not elicit any antibody response against any of the four polysaccharides in B10.BR H-2^{k}, whereas N19 conjugates did. Remarkably, N19 conjugates elicited substantial antibody responses after the first dose in all tested mouse strains against the four polysaccharides with little exceptions in the less immunogenic strains. Most mice of different genetic backgrounds used in this study produced antibodies to the four polysaccharides, indicating a lack of any apparent genetic restriction of immune response upon immunization with N19-conjugates.

As shown in Figure 14, in agreement with IgG responses measured by ELISA, also bactericidal titers obtained with N19 and CRM conjugates were higher in BALB/c H-2^{d} recipients. We observed that N19 conjugates induced higher bactericidal titers than CRM conjugates against the four polysaccharides in all tested strains, except in BALB/B mice against MenA. The evaluation of the functional activity of the produced antibodies by serum bactericidal assay confirmed furthermore the better carrier effect of N19, compared to CRM.

### 4. Enzyme-linked immunosorbent assay (ELISA) protocols.

### 4.1 Meningococcal serogroup A, C, W-135 and Y polysaccharide-specific IgG.

Titration of MenA, MenC, MenW and MenY specific immunoglobulins G (IgG) was performed on individual sera from each mouse according to the assays already described [297]. Nunc Maxisorp 96-well flat-bottom plates were coated overnight at 4°C separately with 5 µg/ml of purified *N. meningitidis* serogroup A, C, W or Y polysaccharides in the presence of 5 µg/ml methylated human serum albumin. The plates were washed three times with PBS containing 0.33% Brij-35 (PBS-Brij), then saturated with 200 µl/ well of PBS containing 5% FCS and 0.33% Brij-35 (PBS-FCS-Brij) for 1h at RT. Single sera were diluted in PBS-FCS-Brij and titrated against the four polysaccharides separately. Plates were incubated overnight at 4 °C. On the following day, plates were washed with PBS-Brij, alkaline phosphatase conjugated goat anti-mouse IgG (Sigma Chemical Co., SA Louis, Mo.) diluted in PBS-FCS-Brij was added and plates were incubated 2 hours at 37°C. Bound antibodies were revealed using 1mg/ml p-nitrophenyl-phosphate (Sigma Chemical Co., SA Louis, Mo.) in diethanolamine solution. After 20 min incubation, the absorbance was read out at 405 nm. Pre-immunization values gave consistently an OD value below 0.1. The results were expressed as titers relative to an in-house reference serum by parallel line analysis, to minimize plate-to plate variation. IgG titers were calculated by using Reference Line Assay [298] and expressed as the logarithm of EU/ ml.

### 4.2 Meningococcal serogroup A and C polysaccharide-specific isotype IgG1/IgG2a

To measure anti-MenA and anti-MenC specific IgG1 and IgG2a antibodies, plates were coated overnight at 4°C with 5µg of methylated human serum albumin/ml and 5 µg of purified MenA or MenC per ml in PBS as described above for IgG ELISA. The plates were then washed and blocked with PBS-FCS-Brij for 1 h at RT. Serum samples were diluted in PBS-FCS-Brij across two plates in parallel starting from 1:100 and incubated for 2 h at 37°C. Biotin-conjugated goat anti-mouse IgG1 or IgG2a antibodies (Southern Biotechnology Associates, Inc.) were added. After 2 h incubation at 37°C horseradishperoxidase-conjugated streptavidin (DAKO) was added to the wells, and the plates were incubated for 1 h at 37°C. The plates were developed with the substrate O-phenylenediamine dihydrochloride (Sigma). Titers were calculated as the reciprocal of the serum dilution at which the OD 0.5 (450 nm).

### 4.3 N19-, TT-, HA-, or CRM-, DT-specific IgG antibodies.

Titration of N19, CRM197 carrier proteins and its parent proteins, therein tetanus toxoid (TT), haemophilus influenzae (HA) and diphtheria toxoid (DT) was performed on pooled sera as described previously [299, 300]. Briefly, 96-well plates (Nunc Maxisorp) were coated overnight at 4°C with 200 µl of a PBS solution containing separately 2 µg/ml of N19, TT, HA or CRM197 or 5 µg/ml of DT antigen. The plates were then washed and blocked with PBS-BSA 1% for 1 h at 37°C. Serum samples were diluted in PBS-BSA 1%- Tween20 0.05% across the plate starting from 1:100 and incubated for 2 h at 37°C. Alkaline phosphatase conjugated goat anti-mouse IgG and p-nitrophenyl-phosphate were used for detection. The presence of antigen-specific antibodies was revealed as described above. The results were expressed as titers relative to an in-house reference serum by parallel line analysis, to minimize plate-to plate variation.

### 4.4 Avidity of meningococcal serogroup A and C IgG antibodies.

The avidity of meningococcal group A and C specific IgG antibodies was assessed by ELISA elution assay of pooled sera using 75 mM of ammonium thiocyanate [NH₄SCN] as chaotropic agent, according to the well-established method [301, 302]. Assay validation included the assessment of antigen stability following incubation with 4 M NH₄SCN [303]. Nunc Maxisorp 96-well flat-bottom plates were coated overnight at 4°C with 5 µg/ ml of purified *N. meningitidis* serogroup A and C polysaccharides separately. The solution was aspirated and the wells were washed three times with PBS-Brij and blocked for 1 h at room temperature with blocking buffer (PBS-FCS-Brij). The plates were washed with wash buffer (PBS-Brij). Test and reference sera were diluted in dilution buffer PBS-FCS-Brij and duplicate twofold serial dilutions in one microplate were prepared. After 2 h incubation at 37°C, the plates were washed three times. Serum samples in one of the duplicate were incubated 15 minutes at room temperature with 75 mM NH4SCN in serum dilution buffer PBS-FCS-Brij, whereas the other duplicate was incubated with diluting buffer alone. After washing, the plates were incubated with alkaline phosphatase conjugated goat anti-mouse IgG antibodies (Sigma Chemical Co., SA Louis, Mo.) as in the above-mentioned ELISA assay. The amount of antibodies remaining bound to the plate after elution with 75 mM NH4SCN was calculated in ELISA units by reference to standard ELISA curves, corresponding to 100% bound antibodies. High-avidity IgG titers were represented in % of bound antibodies in function of the time.

### 5. Serum bactericidal assay against meningococcal strains A, C, W and Y.

The method used for measurement of bactericidal antibody titers has been previously described *(94). N. meningitidis* serogroup A (strain F8238), C (strain 11), W (strain 240070) or Y (strain 240539) target strains were grown overnight at 37°C with 5% CO₂ on chocolate agar plates (starting from a frozen stock). Colonies with an absorbance of 0.05-0.1 at 600 nm were suspended in 7ml Mueller Hinton broth containing 0.25% glucose and incubated shaking for 1.5 hours at 37 °C with 5% CO₂ to reach an absorbance of ~ 0.24-0.4 at 600nm. The bacterial cell suspensions were diluted in GBSS buffer (Gey's balanced salt solution) (SIGMA) and 1% BSA (assay buffer) to yield approximately 10⁵ CFU/ ml. Heat-inactivated (56 °C for 30 min) single or pooled serum samples (50 µl) were diluted serially diluted twofold (reciprocal starting dilution of 4) in buffer in 96-well flat-bottom tissue culture-treated plates (Costar, Inc., Cambridge, Mass.). Equal volumes of cell suspensions and pooled baby rabbit complement (25%) were gently mixed, and 25 µl was added to serially diluted sera. The final volume in each well was 50µl. Controls included (i) bacteria-complement-buffer (complement-dependent control) and (ii) heat-inactivated test serum-bacteria-buffer (complement-independent control). Immediately after the addition of the baby rabbit complement, 10 µl of the controls were plated on Mueller-Hinton agar plates by the tilt method (time zero, t0). The microtiter plates were incubated for all serogroup target strains at 37°C for 1 h with 5% CO₂. After incubation, 10 µl of each sample were plated on Mueller-Hinton agar plates as spots, whereas 10 µl of the controls were plated by the tilt method (time one, t1). Agar plates were incubated for 18 h at 37°C with 5% CO₂, and the colonies corresponding to t0 and t1 were counted. Colonies at t1 were a control of eventual toxicity of the complement or the serum and has to be 1.5 times colonies at t0. The bactericidal titers were expressed as the reciprocal serum dilution yielding ≥50% killing compared to the number of target cells present before incubation with serum and complement (t0). Titers were considered reliable if at least two following dilutions yield ≥90% bacterial killing.

Student's t test (2 tails) was used to compare antibody titers between groups and at different times. A P value of < 0.05 was considered as statistically significant.

### 6. Cell-mediated immune responses.

### 6.1 In vitro proliferation assay with N19- epitopes, N19 or N19- conjugates of BALBc mice primed with N19-MenACWY.

To assess whether the immunization with N19-conjugates primed for carrier-epitope specific T cells, spleens from mice immunized two or three times with tetravalent N19-MenACWY (~6 or 2 µg of protein/ dose) as described above were removed 10 days after the last immunization and tested for their capacity to proliferate following *in vitro* stimulation with single peptides constituting N19 or N19 free or conjugated [304]. The purified N19 employed in this assay did not contain detectable LPS, which could have possibly interfered. Spleens of each mouse group were pooled and dispersed manually. Once washed and counted, cells were cultured at a density of 5 x 10⁵ cells per well in RPMI (GIBCO BRL Life Technologies) supplemented with 25 mM HEPES buffer, 100 U/ ml penicillin, 100 µg/ ml streptomycin, 50 µM 2-mercaptoethanol, 0.15mM L-glutamine, sodium pyruvate, vitamins, sodium pyruvate and a cocktail of non-essential amino acids (GIBCO BRL Life Technologies 1% of a 100 x stock) and 5% fetal calf serum (Hyclone) in flat-bottom 96-well cell culture plates (Corning NY). The cells were cultured in triplicate in the presence of the individual peptides from 0.12 to 30 µM per well (two or threefold dilutions) (~ 0.15- 50 µg/ ml) or of free or conjugated N19 from 0.004 to 1 µM diluted in the same medium were added to triplicate wells to give a total of 200 µl per well. Controls were run with complete culture medium or 10 µg/ ml Concanvalin A, to demonstrate the proliferative capacity of the cells. Plates were incubated at 37°C in 5% CO₂. After five days, cells were pulsed with 0.5 µCi of [³H] thymidine (Amersham Biosciences 1mCi/ ml stock) per well for additional 18 h and harvested with Filtermate Harvester and counted in a liquid scintillation counter (Packard Bioscience). Results of proliferative assays were expressed as stimulation index (SI), calculated by the ratio of counts per minute (cpm) in experimental cultures with the stimulus to counts per minute of control cultures (background) without stimulus. Triplicates of cultures were run in parallel. An SI >2 was considered positive.

To determine whether the strong T helper effect of N19 in the mouse system was mediated by any of the CD4⁺ epitopes originally included in N19, T-cell proliferation of splenocytes from BALB/c mice primed two or three times with N19-MenACWY (6 µg of N19/ dose) was assessed. Spleen cells were stimulated *in vitro* with different concentrations of N19 peptides or with whole N19, either free or conjugated to the polysaccharides. As shown in Figure 15, lymphocytes proliferated substantially in the presence of free or conjugated N19. We observed also T cell proliferation with P23TT peptide consistently in all our experiments (Figures 15 and 16). Within the other tested peptides we observed a T-cell proliferation induced by P30TT, P32TT, HA and HBsAg, even though only in the presence of higher concentrations. When the assays were carried out with C57BL/6 mice, neither of the epitopes stimulated lymphocyte proliferation and N19 stimulated cells only at the highest concentration.

Furthermore we measured N19-specific T cell activation in congenic strains of mice to investigate if there was any MHC-restriction pattern. The activation was analyzed *in vitro* by measuring proliferative responses of spleen cells of mice with different genetic background in the presence of different concentrations of N19, either 1) free or 2) conjugated to the polysaccharides, or with 3) single N19 constituting peptides or with 4) free polysaccharide components. We observed that free N19 induced T cell activation in all strains, but N19 conjugates resulted in differential proliferative responses in the tested strains (Figure 17). Evaluating the influence of the background genes (BALB or B10) on H-2 responses, we observed that mice of H-2^{d} haplotype generated T cells specific for different epitopes. A T cell recall of P23TT epitope was generated in two genetically unrelated mice (BALB/c H-2^{d} and B10.BR H-2^{k}). On the other hand congenic mice (BALB or B10) with different H-2 haplotypes generated different epitope-specific T cell proliferation suggesting that genetic factors outside the MHC complex also influence the response. However, mice with the same genetic background (BALB) generated T-cells reactive for P30TT epitope. Overall, despite the fact that the peptides differed in their level of H-2 restriction, all strains were able to mount a good antibody response against all four polysaccharides with N19-conjugates. Moreover, we observed that any of the four polysaccharides were able to induce a proliferation in any tested strain, indicating that they are T-cell independent antigen and conjugation to a carrier protein does not interfere with their characteristics, such as the capability to induce polysaccharide-specific T cell activation.

### 6.2 Assessment for murine epitope- specific T-cell proliferation: immunizations protocol and proliferation assay.

Synthetic peptides (P2TT, P21TT, P23TT, P30TT, P32TT, HA and HBsAg) with 95% purity were purchased from Primm s.r.1. (Italy). Groups of three BALB/c mice were immunized subcutaneously at the base of the tail with 50 µl volume per mouse containing 50 µg of a single peptide (P2TT, P30TT, P23TT, P32TT, HA, HBsAg) or N19 emulsified in complete Freund's adjuvant (CFA). Seven days later, mice were killed, inguinal and periaortic lymph nodes were removed and pooled form mice within each group, and a single-cell suspension was prepared. The cells were cultured at a density of 3 x 10⁵ cells per well in complete medium (supplemented RPMI as described above for spleen cells) in flat-bottom 96-well cell culture plates (Costar Corp., Cambridge, Mass.). N19 or homologous peptide diluted in the same medium were added to triplicate wells of single mouse or pooled cultured cells at three different concentrations (15, 7.5 and 3.75 mM of all the peptides and 10, 1 and 0.1 µg/ ml of N19). After five days incubation at 37°C at 5% CO₂, cells were pulsed with 0.5 µCi [³H] thymidine for 16 h and then harvested as described above. A non-related peptide CH60 (*in silico* predicted to bind HLA-A2) derived from surface protein *Chlamydia pneumoniae* as employed as negative control in these experiments.

Figure 18 shows that immunization of BALB/c mice with individual peptides resulted in T cell responses specific for P23TT, HA, HBsAg peptides and N19, but not for the un-related CH60 peptide. Mice immunized with P32TT failed to respond to the same peptide. Cells from adjuvant control mice proliferated in response to ConA but not in response to any peptide or N19, thereby demonstrating that the peptides were not mitogenic. In spite of being human epitopes, these findings may explain the strong carrier effect of N19 also in the mouse system.

It will be understood that the invention has been described by way of example only and modifications may be made whilst remaining within the scope of the invention.

### REFERENCES

[1] Armand et al. (1982) J. Biol. Stand 10:335-339.
[2] Cadoz et al. (1985) Vaccine 3:340-342.
[3] MMWR (1997) 46(RR-5) 1-10.
[4] Peltola (2000) Clin Microbiol Rev 13:302-317
[5] Wuorimaa & Kayhty (2002) Scand J Immunol 56:111-129
[6] Balmer et al. (2002) J Med Microbiol 51:717-722
[7] Del Giudice (1992) Curr Opin Immunol 4:454-459
[8] Eltinger (1990) Science 249:423-425
[9] Alexander et al. (2000) J Immunol 164:1625-1633
[10] WO99/55730
[11] Falugi et al. (2001) Eur J Immunol 31:3816-3824
[12] Demotz, S., et al. (1993) Eur J Immunol 23:425-32*.*
[13] Ho, P. C., et al. (1990) Eur J Immunol 20:477-83.
[14] Sinigaglia, F., et al. (1988) Nature 336:778-80.
[15] Panina-Bordignon, P., et al. (1989) Eur J Immunol 19:2237-42.
[16] O'Sullivan, D., et al. (1991) J Immunol 147:2663-9.
[17] Falugi, F., et al. (2001) Eur. J Immunol. 31:3816-24.
[18] Greenstein, J. L., et al. (1992) J Immunol 148:3970-7.
[19] Rothbard, J. B., et al. (1988) Cell 52:515-23.
[20] WO02/3477
[21] WO03/093306
[22] WO04/041157
[23] WO2005002619
[24] PCT/IB2004/003366
[25] WO03/007985
[26] Vaccine (ed Plotkin et al) Fourth Edition ISBN 0- 7216- 9688-0
[27] Wessels et al. (1990) J Clin Invest 86:1428-33.
[28] Wessels et al. (1989) Infect Immun 57:1089-94.
[29] WO03/080678
[30] Ravenscroft et al. (1999) Vaccine 17:2802-2816.
[31] Costantino et al. (1999) Vaccine 17:1251-1263.
[32] Lei et al. (2000) Dev Biol (Basel) 103:259-264.
[33] WO00/38711; US patent 6,146,902.
[34] Lees et al. (1996) Vaccine 14:190-198.
[35] WO95/08348.
[36 ] WO98/4272
[37] US patent 4,882,317
[38] US patent 4,695,624
[39] Mol. Immunol., 1985, 22, 907-919
[40] EP-A-0208375
[41] WO00/10599
[42] Gever et al., Med. Microbiol. Immunol, 165 : 171-288 (1979).
[43] US patent 4,057,685.
[44] US patents 4,673,574; 4,761,283; 4,808,700.
[45] US patent 4,459,286.
[46] US patent 4,965,338
[47] US patent 4,663,160.
[48] US patent 4,761,283
[49] US patent 4,356,170
[50] Lei et al. (2000) Dev Biol (Basel) 103:259-264.
[51] WO00/38711; US patent 6,146,902.
[52] WO99/24578
[53] WO99/36544
[54] WO99/57280
[55] WO00/22430.
[56] Tettelin et al. (2000) Science 287:1809-1815
[57] WO96/29412
[58] Pizza et al. (2000) Science 287:1816-1820
[59] WO01/52885
[60] Bjune et al. (1991) Lancet 338(8775):1093-96
[61] Fukasawa et al. (1999) Vaccine 17:2951-2958.
[62] Rosenqvist et al. (1998) Dev. Biol. Stand. 92:323-333.
[63] Costantino et al. (1992) Vaccine 10:691-698.
[64] Costantino et al. (1999) Vaccine 17:1251-1263.
[65] WO03/007985.
[66] WO00/66791
[67] WO01/649
[68] WO01/649
[69] WO03/020756
[70] WO2004/032958
[71] WO2004/048404.
[72] WO98/18931
[73] WO98/18930
[74] US Patent 6,699,703
[75] US Patent 6,800,744
[76] WO97/4330
[77] WO97/37026
[78] Watson (2000) Pediatr Infect Dis J 19:331-332.
[79] Rubin (2000) Pediatr Clin North Am 47:269-285, v.
[80] Jedrzejas (2001) Microbiol Mol Biol Rev 65:187-207.
[81] WO02/22167.
[82] Paoletti et al., (1990) J Biol Chem 265:18278-83.
[83] Wessels et al., (1990) J Clin Invest 86:1428-33.
[84] Baker et al., (2004) J Infect Dis 171:879-84.
[85] WO02/34771
[86] WO2005/032582
[87] WO02/094851
[88] Dale, Vaccine (1999) 17:193-200
[89] Dale, Vaccine 14(10): 944-948
[90] Dale (1999) Infect Dis Clin North Am 13:227-43, viii.
[91] Ferretti et al. (2001) PNAS USA 98: 4658-4663.
[92] WO02/18595
[93] WO99/58562
[94] McMichael (2000) Vaccine 19 Suppl 1:S101-107.
[95] Gustafsson et al. (1996) N. Engl. J. Med 334:349-355.
[96] Rappuoli et al. (1991) TIBTECH 9:232-238.
[97] Kuroda et al. (2001) Lancet 357(9264):1225-1240; see also pages 1218-1219.
[98] WO00/56360
[99] Infect Immun. 2001 May; 69(5): 3510-3515
[100] WO03/093306
[101] Schuchat (1999) Lancer 353(9146):51-6
[102] W02004/041157
[103] WO2005/002619
[104] Zhu et al., Vaccine (2004) 22:660 - 669
[105] Price et al., Infection and Immunity (2004) 71(1):277-283)
[106] Plante et al., J Infectious Disease (2000) 182:848 - 855)
[107] WO02/079243
[108] WO00/37494
[109] WO03/049762
[110] WO031068811
[111] WO99/28475.
[112] US Patent 6,756,361
[113] Covacci & Rappuoli (2000) J. Exp. Med. 19:587-592.
[114] WO93/18150.
[115] WO99/53310
[116] Covacci et al. (1993) Proc. Natl. Acad. Sci. USA 90: 5791-5795.
[117] Tummuru et al. (1994) Infect. Immun. 61:1799-1809.
[118] Marchetti et al. (1998) Vaccine 16:33-37.
[119] Telford et al. (1994) J. Exp. Med. 179:1653-1658.
[120] Evans et al. (1995) Gene 153:123-127.
[121] WO96/01272 & WO96/01273, especially SEQ ID NO:6.
[122] WO97/25429.
[123] WO98/04702.
[124] Infect Immun. 2002 August; 70(8):4414
[125] J Toxicol Clin Toxicol (2001) 39:85-100.
[126] Demicheli et al. (1998) Vaccine 16:880-884.
[127] Stepanov et al. (1996) J Biotechnol 44:155-160.
*[128]* Infect Immun. 2003 Jan; 71(1):374-383.
[129] Infect Immun. 1999 Oct; 67(10):5395
[130] Infect Immun. 1997 Nov; 65(11):4476-4482
[131] Infect Immun. 2004 October; 72(10): 6148
[132] Proc Natl Acad Sci USA 2004 Aug 24; 101 (34):12652
[133] Infect Immun. 2004 July; 72(7):3829
[134] Biochim Biophys Acta. 2004 Nov 1;1702(2):145
*[135]* J Autoimmun. 1989 Jun; 2 Suppl:81
[136] WO02/02606
[137] Kalman et al. (1999) Nature Genetics 21:385-389.
[138] Read et al. (2000) Nucleic Acids Res 28:1397-406.
[139] Shirai et al. (2000) J. Infect. Dis. 181 (Suppl 3):S524-S527.
[140] WO99/27105.
[141] WO00/27994.
[142] Infect Immun. 2003 Oct 7; 71(10):5498-504
[143] Infect Immun. 2001 May; 69(5):3323-3334
[144] J Clin Microbiol. 1999 Dec; 37(12):3997
[145] Ross et al. (2001) Vaccine 19:4135-4142.
[146] Anderson (2000) Vaccine 19 Suppl 1:S59-65.
[147] Kahn (2000) Curr Opin Pediatr 12:257-262.
[148] Crowe (1995) Vaccine 13:415-421.
[149] J Gen Virol. 2004 Nov; 85(Pt 11):3229
[150] Sutter et al. (2000) Pediatr Clin North Am 47:287-308.
[151] Zimmerman & Spann (1999) Am Fam Physician 59:113-118, 125-126.
[152] Bell (2000) Pediatr Infect Dis J 19:1187-1188.
[153] Iwarson (1995) APMIS 103:321-326.
[154] Gerlich et al. (1990) Vaccine 8 Suppl:S63-68 & 79-80.
[155] Houghton et al., Hepatology (1991) 14:381
[156] Hsu et al. (1999) Clin Liver Dis 3:901-915*.*
[157] Dreesen (1997) Vaccine 15 Suppl:S2-6.
[158] MMWR Morb Mortal Wkly Rep 1998 Jan 16;47(1):12, 19.
[159] WO2004/92360
[160] US Patent 5,378,814
[161] US Patent 6,333,164
[162] WO00/15255
[163] US Patent Application 20020007173.
[164] US Patent 5,693,522.
[165] Moingeon (2001) Vaccine 19:1305-1326.
[166] Rosenberg (2001) Nature 411:380-384.
[167] Dermine, S. et al, "Cancer Vaccines and Immunotherapy," British Medical Bulletin, 2002, 62, 149-162
[168] Espinoza-Delgado I., "Cancer Vaccines," The Oncologist, 2002, 7(suppl3):20-33
[169] Davis, I.D. et al., "Rational approaches to human cancer immunotherapy," Journal of Leukocyte Biology, 2003, 23:3-29
[170] Van den Eynde B, et al., "New tumor antigens recognized by T cells", Curr. Opin. Immunol., 1995, 7:674-81
[171] Rosenberg SA, "Cancer vaccines based on the identification of genes encoding cancer regression antigens, Immunol. Today, 1997, 18:175-82
[172] Offringa R et al., "Design and evalutation of antigen-specific vaccination strategies against cancer", Current Opin. Immunol., 2000, 2:576-582
[173] Rosenberg SA, "A new era for cancer imunotherapy based on the genes that encode cancer antigens," Immunity, 1999, 10:281-7
[174] Sahin U et al., "Serological identification of human tumor antigens," Curr. Opin. Immunol., 1997, 9:709-16
[175] Old LJ et al., "New paths in human cancer serology," J. Exp. Med., 1998, 187:1163-7
[176] Chaux et al., "Identification of MAGE-3 epitopes presented by HLA-DR molecules to CD4(+) T lymphocytes," J. Exp. Med., 1999, 189:767-78
[177] Gold P, et al., "Specific carcinoembryonic antigens of the human digestive system," J. Exp. Med., 1965, 122:467-8
[178] Livingston PO, et al., Carbohydrate vaccines that induce antibodies against cancer: Rationale," Cancer Immunol. Immunother., 1997, 45:1-6
[179] Livingston PO, et al., Carbohydrate vaccines that induce antibodies against cancer: Previous experience and future plans," Cancer Immunol. Immunother., 1997, 45:10-9
[180] Taylor-Papadimitriou J, "Biology, biochemistry and immunology of carcinoma-associated mucins," Immunol. Today, 1997, 18:105-7
[181] Zhao X-J et al., "GD2 oligosaccharide: target for cytotoxic T lymphocytes," J. Exp. Med., 1995, 182:67-74
[182] Theobald M, et al., "Targeting p53 as a general tumor antigen," Proc. Natl. Acad. Sci. USA, 1995, 92:11993-7
[183] Gaudernack G, "T cell responses against mutant ras: a basis for novel cancer vaccines," Immunotechnology, 1996, 2:3-9
[184] WO91/02062
[185] US Patent 6,015,567
[186] WO01/08636
[187] WO96/30514
[188] US Patent 5,846,538
[189] US Patent 5,869,445
[190] Ingram (2001) Trends Neurosci 24:305-307.
[191] US Patent 6,884,435.
[192] WO98/20734.
[193] WO03/009869
[194] WO01/30390.
[195] Vaccine Design... (1995) eds. Powell & Newman. ISBN: 030644867X. Plenum.
[196] WO00/23105.
[197] WO90/14837.
[198] Podda (2001) Vaccine 19:2673-80.
[199] Frey et al. (2003) Vaccine 21:4234-7.
[200] US Patent 6,299,884.
[201] US Patent 6,451,325.
[202] Allison & Byars (1992) Res Immunol 143:519-25.
[203] Hariharan et al. (1995) Cancer Res 55:3486-9.
[204] US patent 5,057,540.
[205] WO96/33739.
[206] EP-A-0109942.
[207] WO96/11711.
[208] WO00/07621.
[209] Barr et al. (1998) Advanced Drug Delivery Reviews 32:247-271.
[210] Sjolanderet et al. (1998) Advanced Drug Delivery Reviews 32:321-338.
[211] Niikura et al. (2002) Virology 293:273-280.
[212] Lenz et al. (2001) J Immunol 166:5346-5355.
[213] Pinto et al. (2003) J Infect Dis 188:327-338.
[214] Gerber et al. (2001) Virol 75:4752-4760.
[215] WO03/024480
[216] WO03/024481
[217] Gluck et al. (2002) Vaccine 20:B 10-B16.
[218] EP-A-0689454.
[219] Johnson et al. (1999) Bioorg Med Chem Lett 9:2273-2278.
[220] Evans et al. (2003) Expert Rev Vaccines 2:219-229.
[221] Meraldi et al. (2003) Vaccine 21:2485-2491.
[222] Pajak et al. (2003) Vaccine 21:836-842.
[223] Kandimalla et al. (2003) Nucleic Acids Research 31:2393-2400.
[224] WO02/26757.
[225] WO99/62923.
[226] Krieg (2003) Nature Medicine 9:831-835.
[227] McCluskie et al. (2002) FEMS Immunology and Medical Microbiology 32:179-185.
[228] WO98/40100
[229] US patent 6,207,646.
[230] US patent 6,239,116.
[231] US patent 6,429,199.
[232] Kandimalla et al. (2003) Biochemical Society Transactions 31 (part 3):654-658.
[233] Blackwell et al. (2003) J Immunol 170:4061-4068.
[234] Krieg (2002) Trends Immunol 23:64-65.
[235] WO01/95935.
[236] Kandimalla et al. (2003) BBRC 306:948-953.
[237] Bhagat et al. (2003) BBRC 300:853-861.
[238] WO03/035836.
[239] WO95/17211.
[240] WO98/42375.
[241] Beignon et al. (2002) Infect Immun 70:3012-3019.
[242] Pizza et al. (2001) Vaccine 19:2534-2541.
[243] Pizza et al. (2000) Int J Med Microbiol 290:455-461.
[244] Scharton-Kersten et al. (2000) Infect Immun 68:5306-5313.
[245] Ryan et al. (1999) Infect Immun 67:6270-6280.
[246] Partidos et al. (1999) Immunol Lett 67:209-216.
[247] Peppoloni et al. (2003) Expert Rev Vaccines 2:285-293.
[248] Pine et al. (2002) J Control Release 85:263-270.
[249] Domenighini et al. (1995) Mol Microbiol 15:1165-1167*.*
[250] WO03/011223.
[251] WO99/40936
[252] Matsui M. et al. (2004) J. Virol 78: 9093.
[253] WO99/44636.
[254] Lillard JW et al., (2003) Blood Feb 1; 101 (3):807-14. Epub 2002 Sep 12.
[255] Singh et al] (2001) J Cont Release 70:267-276.
[256] WO99/27960.
[257] US patent 6,090,406
[258] US patent 5,916,588
[259] EP-A-0626169.
[260] WO99/52549.
[261] WO01/21207.
[262] WO01/21152.
[263] Andrianov et al. (1998) Biomaterials 19:109-115*.*
[264] Payne et al. (1998) Adv Drug Delivery Review 31:185-196.
[265] Stanley (2002) Clin Exp Dermatol 27:571-577.
[266] Jones (2003) Curr Opin Investig Drugs 4:214-218.
[267] WO04/60308
[268] WO04/64759.
[269] US 6,924,271.
[270] US2005/0070556.
[271] US 5,658,731.
[272] Wong et al. (2003) J Clin Pharmaco/ 43(7):735-42.
[273] US2005/0215517.
[274] WO02/072012
*[275]* Vaccine Adjuvants: Preparation Methods and Research Protocols (Volume 42 of Methods in Molecular Medicine series). ISBN: 1-59259-083-7. Ed. O'Hagan.
[276] Signorelli & Hadden (2003) Int Immunopharmacol 3(8):1177-86.
[277] W02004/064715.
[278] Cooper (1995) Pharm Biotechnol 6:559-80.
[279] PCT/US2005/022769.
[280] WO2004/87153.
[281] US 6,605,617.
[282] WO02/18383.
[283] WO2004/018455.
[284] WO03/082272.
[285] US patent 5,011,828.
[286] US-6586409.
[287] WO99/11241.
[288] WO94/00153.
[289] WO98/57659.
[290] European patent applications 0835318, 0735898 and 0761231.
[291] Costantino, P., et at (1992). Vaccine 10:691-8.
[292] Costantino, P., et al. (1999) Vaccine 17:1251-63.
[293] Ravenscroft, N., G. et al. (1999) Vaccine 17:2802-16.
[294] WO 03/007985
[295] Porro, M., P. et al. (1985) Mol Immunol 22:907-19.
[296] Goldblatt, D., et al. (1998) J Infect Dis 177:1112-5
[297] Carlone, G. M., et al. (1992) J Clin Microbiol 30:154-9.
[298] Grabowska, K., et al. (2002) J Immunol Methods 271:1-15.
[299] Baraldo, K., et al. (2004) Infect Immun 72:4884-7.
[300] Mawas, F., et al. (2004) J Infect Dis 190:1177-82.
[301] Goldblatt, D., et al. (1998) J Infect Dis 177:1112-5.
[302] Granoff, D. M., et al. (1998) Clin Diagn Lab Immunol 5:479-85.
[303] Schallert, N., et al. (2002) Eur J Immunol 32:752-60*.*
[304] Valmori, D., et al. (1992) J Immunol 149:717-21.

### SEQUENCE LISTING

***SEQ ID NO: 1 (P23TT)***
   1 VSIDKFRIFC KANPK
***SEQ ID NO: 2 (P32TT)***
   1 LKFIIKRYTP NNEIDS
***SEQ ID NO: 3 (P21TT)***
   1 IREDNNITLK LDRCNN
***SEQ ID NO: 4 (PfCs)***
   1 EKKIAKMEKA SSVFNVVN
***SEQ ID NO: 5 (P30TT)***
   1 FNNFTVSFWL RVPKVSASHL E
***SEQ ID NO: 6 (P2TT)***
   1 QYIKANSKFI GITE
***SEQ ID NO: 7 (HBVnc)***
   1 PHHTALRQAI LCWGELMTLA
***SEQ ID NO: 8 (HA)***
   1 PKYVKQNTLK LAT
***SEQ ID NO: 9 (HBsAg)***
   1 FFLLTRILTI PQSLD
***SEQ ID NO: 10 (MT)***
   1 YSGPLKAEIA QRLEDV
***SEQ ID NO: 11 (N19)*** 
Met Gly Gly Ser His His His His His His Gly Met Ala Ser Met Asp
1               5                   10                  15

Tyr Lys Asp Asp Asp Asp Ile Glu Gly Arg Lys Gly Val Ser Ile Asp
            20                  25                  30

Lys Phe Arg Ile Phe Cys Lys Ala Asn Pro Lys Lys Gly Leu Lys Phe
        35                  40                  45

Ile Ile Lys Arg Tyr Thr Pro Asn Asn Glu Ile Asp Ser Lys Gly Ile
    50                  55                  60

Arg Glu Asp Asn Asn Ile Thr Leu Lys Leu Asp Arg Cys Asn Asn Lys
 
65                  70                  75                  80

Gly Glu Lys Lys Ile Ala Lys Met Glu Lys Ala Ser Ser Val Phe Asn
                85                  90                  95

Val Val Asn Ser Lys Gly Phe Asn Asn Phe Thr Val Ser Phe Trp Leu
            100                 105                 110

Arg Val Pro Lys Val Ser Ala Ser His Leu Glu Lys Gly Gln Tyr Ile
        115                 120                 125

Lys Ala Asn Ser Lys Phe Ile Gly Ile Thr Glu Lys Gly Gly Ser Pro
    130                 135                 140

His His Thr Ala Leu Arg Gln Ala Ile Leu Cys Trp Gly Glu Leu Met
145                 150                 155                 160

Thr Leu Ala Lys Gly Ser Pro Lys Tyr Val Lys Gln Asn Thr Leu Lys
                165                 170                 175

Leu Ala Thr Lys Gly Ser Phe Phe Leu Leu Thr Arg Ile Leu Thr Ile
            180                 185                 190

Pro Gln Ser Leu Asp Lys Gly Tyr Ser Gly Pro Leu Lys Ala Glu Ile
        195                 200                 205

Ala Gln Arg Leu Glu Asp Val Lys Gly Ser Val Ser Ile Asp Lys Phe
    210                 215                 220

Arg Ile Phe Cys Lys Ala Asn Pro Lys Lys Gly Leu Lys Phe Ile Ile
225                 230                 235                 240

Lys Arg Tyr Thr Pro Asn Asn Glu Ile Asp Ser Lys Gly Ile Arg Glu
                245                 250                 255

Asp Asn Asn Ile Thr Leu Lys Leu Asp Arg Cys Asn Asn Lys Gly Glu
            260                 265                 270

Lys Lys Ile Ala Lys Met Glu Lys Ala Ser Ser Val Phe Asn Val Val
        275                 280                 285

Asn Ser Lys Gly Phe Asn Asn Phe Thr Val Ser Phe Trp Leu Arg Val
    290                 295                 300

Pro Lys Val Ser Ala Ser His Leu Glu Lys Gly Gln Tyr Ile Lys Ala
305                 310                 315                 320

Asn Ser Lys Phe Ile Gly Ile Thr Glu Lys Gly Gly Ser Pro His His
                325                 330                 335

Thr Ala Leu Arg Gln Ala Ile Leu Cys Trp Gly Glu Leu Met Thr Leu
            340                 345                 350

Ala Lys Gly Ser Pro Lys Tyr Val Lys Gln Asn Thr Leu Lys Leu Ala
        355                 360                 365

Thr Lys Gly Ser Phe Phe Leu Leu Thr Arg Ile Leu Thr Ile Pro Gln
    370                 375                 380

Ser Leu Asp Lys Gly Ser
385                 390
***SEQ ID NO: 12 (Immunoaffinity tag)***
   1 MDYKDDDD
***SEQ ID NO: 13 (Factor Xa recognition site)***
   1 IEGR

## Claims

1. A composition comprising a combination of two or more monovalent conjugates, wherein each of said two or more monovalent conjugates comprises (i) N19 carrier protein conjugated to (ii) a saccharide antigen from *N. meningitidis* serogroup A, C, W-135, or Y.

2. A multivalent W-135 conjugate comprising two or more antigenically distinct saccharide antigens from *N. meningitidis* serogroups A, C, W-135, or Y, wherein said saccharide antigens are conjugated to N19 carrier protein.

3. A composition comprising two or more of the multivalent conjugates according to claim 2.

4. A composition comprising one or more multivalent conjugates according to claim 2 and one or more monovalent conjugates as defined in claim 1.

5. A composition according to claims 1 or 4, wherein a molecule of said carrier protein in said monovalent conjugate is conjugated to more than one molecule of said saccharide antigen.

6. A composition according to claims 1 or 4, wherein each carrier protein molecule in each monovalent conjugate is conjugated to more than one saccharide antigen molecule.

7. A composition comprising a first conjugate and a second conjugate, wherein said first conjugate comprises a saccharide antigen from *N. meningitidis* serogroup A, C, W-135 , or Y, conjugated to N19 carrier protein; and said second conjugate comprises a saccharide antigen from *N. meningitidis* serogroups A, C, W-135, or Y, conjugated to a carrier protein different from N19.

8. A conjugate or composition according to any previous claim further comprising an adjuvant.

9. A conjugate or composition according to any previous claim for use in therapy.

10. A conjugate or composition according to any previous claim for use in raising an immune response.

11. Use of a conjugate or composition according to any one of claims 1-8 in the manufacture of a medicament for raising an immune response in a patient.

## Patentansprüche

1. Zusammensetzung, die eine Kombination von zwei oder mehreren monovalenten Konjugaten umfasst, wobei jedes der zwei oder mehreren monovalenten Konjugate (i) N19-Trägerprotein umfasst, das mit (ii) einem Saccharid-Antigen von *N. meningitidis* der Serogruppen A, C, W-135 oder Y konjugiert ist.

2. Multivalentes Konjugat, das zwei oder mehrere antigenisch unterschiedliche Saccharid-Antigene der Serogruppen A, C, W-135 oder Y von *N. meningitidis* umfasst, wobei die Saccharid-Antigene mit dem N19 Trägerprotein konjugiert sind.

3. Zusammensetzung, die zwei oder mehrere der multivalenten Konjugate nach Anspruch 2 umfasst.

4. Zusammensetzung, die ein oder mehrere der multivalenten Konjugate nach Anspruch 2 sowie ein oder mehrere der in Anspruch 1 definierten monovalenten Konjugate umfasst.

5. Zusammensetzung nach Anspruch 1 oder 4, wobei ein Molekül des Trägerproteins in dem monovalenten Konjugat mit mehr als einem Molekül des Saccharid-Antigens konjugiert ist.

6. Zusammensetzung nach Anspruch 1 oder 4, wobei jedes Trägerproteinmolekül in jedem monovalenten Konjugat mit mehr als einem Saccharid-Antigenmolekül konjugiert ist.

7. Zusammensetzung, die ein erstes Konjugat und ein zweites Konjugat umfasst, wobei das erste Konjugat ein Saccharid-Antigen der Serogruppen A, C, W-135 oder Y von *N. meningitidis* umfasst, welches mit N19 Trägerprotein konjugiert ist; und das zweite Konjugat ein Saccharid-Antigen der Serogruppen A, C, W-135 oder Y von *N. meningitidis* umfasst, welches mit einem Trägerprotein konjugiert ist, welches sich von N19 unterscheidet.

8. Konjugat oder Zusammensetzung nach einem der vorhergehenden Ansprüche, das außerdem ein Adjuvans umfasst.

9. Konjugat oder Zusammensetzung nach einem der vorhergehenden Ansprüche zur Verwendung bei der Therapie.

10. Konjugat oder Zusammensetzung nach einem der vorhergehenden Ansprüche zur Verwendung bei der Erzeugung einer Immunantwort.

11. Verwendung eines Konjugats oder einer Zusammensetzung nach einem der Ansprüche 1 bis 8 in der Herstellung eines Medikaments zur Erzeugung einer Immunantwort in einem Patienten.

## Revendications

1. Composition comprenant une combinaison de deux conjugués monovalents ou plus, dans laquelle chacun desdits deux conjugués monovalents ou plus comprend (i) une protéine vectrice N19 conjuguée à (ii) un antigène saccharidique provenant du sérogroupe A, C, W-135, ou Y de *N. meningitidis.*

2. Conjugué multivalent comprenant deux antigènes saccharidiques antigéniquement distincts ou plus provenant des sérogroupes A, C, W-135, ou Y de *N*. *meningitidis,* dans lequel lesdits antigènes saccharidiques sont conjugués à la protéine vectrice N19.

3. Composition comprenant deux conjugués multivalents ou plus selon la revendication 2.

4. Composition comprenant un conjugué multivalent ou plus selon la revendication 2 et un conjugué monovalent ou plus selon la revendication 1.

5. Composition selon les revendications 1 ou 4, dans laquelle une molécule de ladite protéine vectrice dans ledit conjugué monovalent est conjuguée à plus d'une molécule dudit antigène saccharidique.

6. Composition selon les revendications 1 ou 4, dans laquelle chaque molécule de protéine vectrice dans chaque conjugué monovalent est conjuguée à plus d'une molécule d'antigène saccharidique.

7. Composition comprenant un premier conjugué et un second conjugué, dans laquelle ledit premier conjugué comprend un antigène saccharidique provenant du sérogroupe A, C, W-135 ou Y de *N*. *meningitidis,* conjugué à une protéine vectrice N19 ; et ledit second conjugué comprend un antigène saccharidique provenant des sérogroupes A, C, W-135, ou Y de *N. meningitidis,* conjugué à une protéine vectrice différente de N19.

8. Conjugué ou composition selon l'une quelconque des revendications précédentes comprenant, en outre, un adjuvant.

9. Conjugué ou composition selon l'une quelconque des revendications précédentes pour son utilisation thérapeutique.

10. Conjugué ou composition selon l'une quelconque des revendications précédentes pour son utilisation pour provoquer une réponse immunitaire.

11. Utilisation d'un conjugué ou d'une composition selon l'une quelconque des revendications 1 à 8 pour la fabrication d'un médicament destiné à provoquer une réponse immunitaire chez un patient.
